# EUROPÄISCHE PATENTSCHRIFT

(11) **EP 2 627 666 B1**
(45) Veröffentlichungstag und Bekanntmachung des Hinweises auf die Patenterteilung: **08.07.2015**
(21) Anmeldenummer: 11791198.2
(22) Anmeldetag: 12.10.2011
(51) Int. Cl.: C07K 14/195, C12N 9/42, C12N 9/78

(54) **NITRILASEN MIT GESTEIGERTE AKTIVITÄT**
NITRILASES WITH IMPROVED ACTIVITY
NITRILASES AYANT UNE ACTIVITE AMELIOREE

(30) Priorität: 12.10.2010 EP 10013548
(43) Veröffentlichungstag der Anmeldung: 21.08.2013
(73) Patentinhaber: c-LEcta GmbH, 04103 Leipzig (DE)
(72) Erfinder: VOGEL, Andreas, 04105 Leipzig (DE); SCHWARZE, Daniel, 07743 Jena (DE); GREINER-STÖFFELE, Thomas, 04279 Leipzig (DE)
(74) Vertreter: Bülle, Jan
(86) Internationale Anmeldenummer: PCT/EP2011/005115
(87) Internationale Veröffentlichungsnummer: WO 2012/048865

(56) Entgegenhaltungen:
- EP-A1- 1 767 624
- WO-A2-2006/069110
- WO-A2-2006/069114
- US-A1- 2009 111 158
- US-A1- 2009 111 162
- US-A1- 2009 325 250
- MARTÍNKOVÁ LUDMILA ET AL: "Biotransformations with nitrilases.", CURRENT OPINION IN CHEMICAL BIOLOGY APR 2010 LNKD- PUBMED:20083424, Bd. 14, Nr. 2, April 2010 (2010-04), Seiten 130-137, XP002619913, ISSN: 1879-0402
- SHIJUN WU ET AL: "Protein engineering of Acidovorax facilis 72W nitrilase for bioprocess development", BIOTECHNOLOGY AND BIOENGINEERING, Bd. 97, Nr. 4, 1. Juli 2007 (2007-07-01), Seiten 689-693, XP55041811, ISSN: 0006-3592, DOI: 10.1002/bit.21289
- SHIJUN WU ET AL: "Protein engineering of nitrilase for chemoenzymatic production of glycolic acid", BIOTECHNOLOGY AND BIOENGINEERING, Bd. 99, Nr. 3, 15. Februar 2008 (2008-02-15), Seiten 717-720, XP55041810, ISSN: 0006-3592, DOI: 10.1002/bit.21643
- CHAUHAN S ET AL: "Purification, cloning, sequencing and over-expression in Escherichia coli of a regioselective aliphatic nitrilase from Acidovorax facilis 72W", APPLIED MICROBIOLOGY AND BIOTECHNOLOGY, SPRINGER VERLAG, BERLIN, DE, Bd. 61, Nr. 2, 16. Januar 2003 (2003-01-16), Seiten 118-122, XP002376999, ISSN: 0175-7598
- BARGLOW KATHERINE T ET AL: "Functional proteomic and structural insights into molecular recognition in the nitrilase family enzymes.", BIOCHEMISTRY 23 DEC 2008 LNKD- PUBMED:19053248, Bd. 47, Nr. 51, 23. Dezember 2008 (2008-12-23), Seiten 13514-13523, XP002619954, ISSN: 1520-4995
- DESANTIS G ET AL: "Creation of a productive, highly enantioselective nitrilase through gene site saturation mutagenesis (GSSM)", JOURNAL OF THE AMERICAN CHEMICAL SOCIETY, AMERICAN CHEMICAL SOCIETY, US, Bd. 125, Nr. 38, 24. September 2003 (2003-09-24), Seiten 11476-11477, XP002385384, ISSN: 0002-7863, DOI: DOI:10.1021/JA035742H

## Beschreibung

Die Erfindung betrifft Nitrilasen mit erhöhter Aktivität und Temperaturstabilität. Nitrilasen werden für die Synthese von Carbonsäuren aus den entsprechenden Nitrilen eingesetzt. Sie zeichnen sich gegenüber chemischen Katalysatoren durch mildere Reaktionsbedingungen aus und werden bevorzugt für regio- und stereoselektive Hydrolysen eingesetzt, bei denen es bisher keine chemische Alternative gibt. Der Einsatz von Nitrilasen zur Synthese von Carbonsäuren ist in der Literatur beschrieben (R. Singh, R. Sharma, N. Tewari, and D. S. Rawat. Chem Biodivers. 3 (12):1279-1287, 2006.)

Um einen kostengünstigen Syntheseprozess realisieren zu können, werden bevorzugt Nitrilasen eingesetzt, die eine hohe spezifische Aktivität und eine lange Prozessstabilität besitzen. Dadurch ist es möglich, eine geringere Katalysatormenge einzusetzen. Eine hohe Prozessstabilität korreliert zumeist mit einer erhöhten Temperaturstabilität der Enzyme. Ein Enzym mit erhöhter Temperaturstabilität hat darüber hinaus den Vorteil, dass der Prozess auch bei höheren Temperaturen effektiv durchgeführt werden kann, was häufig zu noch schnelleren Reaktionszeiten führt.

Nitrilasen sind aus verschiedenen Mikroorganismen isoliert worden, z.B. aus den Gattungen Aspergillus, Arthrobacter, Geobacillus, Fusarium, Norcadia, Rhodococcus, Alcaligenes, Acidovorax, Acinetobacter, Bradyrhizopium, Pseudomonas, Pyrococcus, Bacillus. (R. N. Thuku, D. Brady, M. J. Benedik, and B. T. Sewell. Journal of Applied Microbiology 106 (3):703-727, 2009; C. O'Reilly and P. D. Turner. Journal of Applied Microbiology 95 (6):1161-1174, 2003.).

Die Nitrilase aus Acidovorax facilis wurde erstmals beschrieben in US 6,870,038. Die heterologe Expression des Enzyms in *E. coli* führte zu einer Steigerung der Aktivität pro Biomasse um ca. Faktor 3 (US 6,870,038).

Eine Möglichkeit zur Verbesserung von Enzymen besteht in der Anwendung von Enzym-Engineering. Enzym-Engineering zielt auf die Entwicklung von Varianten eines Ausgangsenzyms mit verbesserten Eigenschaften ab.

Mutationen zur Aktivitätssteigerung einer Nitrilase für die Herstellung von 3-Hydroxy-valerinsäure sind beschrieben in: S. Wu, A. J. Fogiel, K. L. Petrillo, E. C. Hann, L. J. Mersinger, R. DiCosimo, D. P. O'Keefe, A. Ben Bassat, and M. S. Payne. Biotechnol Bioeng. 97 (4):689-693, 2007 und US 7148051. Ein Austausch der Aminosäuren an den Positionen T210 zu A oder C und an F168 zu K, V oder L führte zu einer erhöhten Aktivität. Weitere Mutationen der Acidovorax facilis Nitrilase sind beschrieben in S. Wu, A. J. Fogiel, K. L. Petrillo, R. E. Jackson, K. N. Parker, R. DiCosimo, A. Ben Bassat, D. P. O'Keefe, and M. S. Payne. Biotechnol.Bioeng. 99 (3):717-720, 2008. und US 7198927. Es wird dort eine Aktivitätsteigerung für die Synthese von Glykolsäure aus Glykolonitril für die Einzelmutationen F168 zu V, K, M, T, L201 zu N, Q, K. H, S, T, A, G sowie für einige Varianten mit Kombinationen dieser Einzelmutationen beschrieben. Bei der Variante F168V wurde zudem eine gesteigerte Temperaturstabilität der enzymhaltigen Zellsuspension gegenüber der Zelle, die das Wildtyp-Enzym enhält, festgestellt. Hier wurde die Temperaturstabilität des Biokatalysators innerhalb der intakten E. coli Zelle getestet, die i.d.R. einen günstigen Einfluss auf die Proteinstabilität hat.

US 2009/111158 offenbart ein Verfahren zur Herstellung enzymatischer Katalysatoren mit Nitrilase-Aktivität zur Hydrolyse von Glykolnitril zu Glykolsäure.

WO 2006/069114 betrifft ein Verfahren zur Herstellung von Glykolsäure aus Formaldehyd und Blausäure.

US 2010/240109 offenbart ein Verfahren zur Verbesserung der spezifischen Aktivität enzymatischer Katalysatoren mit Nitrilase-Aktivität bei der Umsetzung von Glykolnitril zu Glykolsäure in wässrigem Medium.

WO 2006/069110 verschiedene Methoden zur enzymatischen Herstellung von Glykolsäure aus Glykolnitril.

K.T. Barglow et al., Biochemistry 2008, 47, 13514-13525 offenbart funktionelle und strukturelle Untersuchungen zur molekularen Erkennung bei Enzymen der Familie der Nitrilasen.

EP 1 767 624 offenbart Proteine mit verbesserter Nitrilhydratase-Aktivität und verbesserter Hitzeresistenz.

L. Martinkova et al., Curr Op in Chem Biol, Bd. 14, Nr. 2, 2010, 130-137 betrifft Untersuchungen zu Biotransformationen mit Hilfe von Nitrilasen.

G. DeSantis et al., J Am Chem Soc 125(83), 2003, 11476-11477 offenbart die Erzeugung produktiver, hochgradig enantioselektiver Nitrilasen mit Hilfe von Sättigungs-Mutagenese (GSSM).

S. Chauvan et al., Appl Microbiol Biotech, Bd. 61, Nr. 2, 2003, 118-122 betrifft die Reinigung, Klonierung, Sequenzierung und Überexprimierung einer regioselektiven aliphatischen Nitrilase aus Acidovorax facilis 72W in E. coli.

US 2009/111162 A1 und US 2009/325250 A1 offenbaren ein Verfahren zur Verbesserung der spezifischen Aktivität eines Enzym-Katalysators mit Nitrilase-Aktivität bei der Umwandlung von Glykolnitril in Glykolsäure unter wässrigen Reaktionsbedingungen.

Der Erfindung liegt die Aufgabe zugrunde, eine Nitrilase mit verbesserten Eigenschaften bereitzustellen. Die Nitrilase sollte sich im Vergleich zu den bekannten Nitrilasen durch eine erhöhte Aktivität auszeichnen, so dass die Einsatzmenge des Enzyms verringert oder die Reaktionszeiten verkürzt werden können. Ferner sollte die Nitrilase eine erhöhte Stabilität, insbesondere eine erhöhte Temperaturstabilität aufweisen, um eine längere Lebensdauer während der durch sie katalysierten Umsetzung zu ermöglichen. Die Umsetzung kann dann mit geringerer Enzymmenge für längere Zeit bzw. mit hoher Reaktionsgeschwindigkeit bei erhöhter Temperatur durchgeführt werden.

Diese Aufgabe wird durch den Gegenstand der Patentansprüche gelöst.

Es wurden überraschend Aminosäure-Positionen in der Protein-Sequenz der Nitrilase aus Acidovorax facilis (Seq ID No: 2) gefunden, die durch Substitution (Austausch) der Aminosäuren an einzelnen Positionen und/oder durch Kombination mehrerer Positionen eine erhöhte Aktivität mit verschiedenen Substraten und/oder eine erhöhte Temperaturstabilität zeigen. Dieser Effekt ist überraschenderweise auch bei den isolierten Enzymen, die sich außerhalb intakter Zellen befinden, zu beobachten. Insbesondere konnten überaschenderweise Aminosäure-Positionen identifiziert werden, an denen Einzelmutationen gleichzeitig zu einer Verbesserung der Aktivität und der Stabilität des Enzyms führen. Ebenfalls überraschenderweise konnten Aminosäure-Positionen identifiziert werden, deren Kombination zu einer synergistischen Verbesserung der relevanten Eigenschaften des Enzyms führen (Kombinierbarkeit der Einzelmutationen).

Infolge der Erhöhung der Aktivität kann die Einsatzmenge des Enzyms verringert oder die Reaktionszeiten verkürzt werden. Beides wirkt sich direkt positiv auf die Synthesekosten aus und hat damit wirtschaftliche Bedeutung. Die verbesserte Stabilität, im speziellen der Temperaturstabilität des Enzyms, ermöglicht eine längere Lebensdauer während der Umsetzung. Die Umsetzung kann daher mit geringerer Enzymmenge für längere Zeit durchgeführt werden oder die Umsetzung kann mit hoher Reaktionsgeschwindigkeit bei erhöhter Temperatur durchgeführt werden. Beide Maßnahmen führen zu einer Reduktion der Synthesekosten.

Im Gen der Nitrilase aus Acidovorax facilis konnten Aminosäure-Positionen identifiziert werden, die zu einer Steigerung der Aktivität und der Stabilität des Enzyms führen. Gezeigt werden konnte dies für die Positionen L9, V63, T70, R94, F168, L194, T208, C250, V305 und D308. Die Substitutionen führten dabei überraschenderweise sowohl zu einer Aktivitätssteigerung bei den Positionen L9, V63, T70, F168, T208, C250 als auch zu einer Erhöhung der Temperaturstabilität an den Positionen L9, V63, T70, R94, F168, C250, V305. Es wurde überraschend gefunden, dass Substitutionen an den einzelnen Positionen kombiniert werden können, wodurch eine weitere Steigerung der Aktivität und Temperaturstabilität erreicht wird.

### Definitionen

### Ausgangsenzym

Mit dem Ausgangsenzym ist die Nitrilase aus Acidovorax facilis gemäß Seq ID 2 gemeint, welches durch die Nukleotidsequenz entsprechend Seq 1 kodiert wird. Das Enzym kann durch heterologe Expression produziert werden. Dabei wird ein Gen mit einer für das Ausgangsenzym kodierenden Nukleotidsequenz in einen entsprechenden Expressiohsvektor eingebaut und in eine Wirtszelle eingebracht. Als Wirtszelle eignen sich unter anderem Stämme der Gattung Escherichia, Bacillus, Saccharomyces, Pichia, Hansenula und Kluyveromyces.

### Substitution

Eine Verbesserung der Enzymeigenschaften wird duch eine veränderte Abfolge der Aminosäuren im Polypeptid des Ausgangsenzyms erreicht. Bei einer Substitution wird eine Aminosäure durch eine andere ersetzt. Es können dabei auch mehrere Aminosäuren entweder nacheinander oder gleichzeitig ersetzt werden.

### Aktivität

Die Aktivität der Nitrilase Enzyme wird durch die Beobachtung der von Ihnen katalysierten Reaktion der Hydrolyse von Nitrilen zu Carbonsäuren gemessen. Dabei werden verschiedene Nitrile, wie 2-Methylglutaronitril (2MG), 1-(Cyanomethyl)cyclohexan-1-carbonitril (CH-dinitril) oder Benzonitril eingesetzt. Eine Enzym Einheit entspricht dabei der Hydrolyse von 1 µmol Nitril in 1 Minute unter definierten Bedingungen und wird in Units (U) angegeben. Die Reaktionsanalyse geschieht in der Regel über GC- oder HPLC-Analytik. Standardbedingungen für die hier beschriebenen Untersuchungen sind: 5 mM 2MG in 100 mM Kaliumphosphatpuffer pH 7.0 wird bei 30°C für 15 min mit einer Nitrilase in Kontakt gebracht in einem Gesamtvolumen von 200 µl. Die Reaktion wird nach dieser Zeit mit 30 µl 1 N Salzsäurelösung gemischt, wodurch die Reaktion gestoppt wird. Die angesäuerte Reaktionslösung wird mit 230 µl Methyl-tert-butylether (MTBE) extrahiert wobei das Nitril und die entstandene Carbonsäure in die organische Phase überführt werden. Die MTBE Phase wird dann mittels Gaschromatographie (GC) analysiert (Säule: ZB-5HT (Phenomenex, Germany), 1 min bei 80°C, auf 130°C mit 20°C/min, 1 min bei 130°C. Die Detektion erfolgt mit einem Flammenionisationsdetektor (FID)).

### Temperaturstabilität

Die Temperaturstabilität wird gemessen in dem eine Enzymlösung in 100 mM Kaliumphosphatpuffer pH 7.0 mit 1 mM DTT für 15 min bei einer definierten Temperatur inkubiert wird. Nach dieser Zeit wird die Enzymlösung für 10 min auf Eis inkubiert. Die Lösung wird zentrifugiert um unlösliche Bestandteile abzutrennen und die Aktivität der überstehenden Lösung gemessen. Die Restaktivität in % wird erhalten in dem die Aktivität nach der Temperaturbehandlung durch die gemessene Aktivität ohne Temperaturbehandlung dividiert wird.

Nitrilasen katalysieren die Hydrolyse von Nitrilen zu Carbonsäuren ohne Zwischenstufe, d.h. die Reaktion verläuft durch Addition zweier Wassermoleküle vollständig bis zur Carbonsäure unter Freisetzung von Ammoniak, ohne dass die Amid-Zwischenstufe isoliert werden kann. Nitrilasen werden systematisch auch als "Nitril-Aminohydrolasen" bezeichnet (E.C. 3.5.5.1).

Die Erfindung betrifft eine Nitrilase umfassend eine Aminosäuresequenz mit mindestens 60% Identität zur Aminosäuresequenz gemäß (Seq ID No: 2), welche im Vergleich zur Nitrilase gemäß (Seq ID No: 2)
(i) eine um mindestens 15% gesteigerte Aktivität bei der Umsetzung eines Nitrils zur entsprechenden Carbonsäure aufweist, wobei das Nitril bevorzugt ausgewählt ist aus der Gruppe bestehend aus 2-Methylglutaronitril, 1-(Cyanomethyl)cyclohexan-1-carbo-nitril und Benzonitril;
   und
(ii) bestimmte Aminosäure-Substitutionen aufweist.

Die Aminosäure-Substitutionen sind in Tabelle 1 zusammengestellt. Besonders bevorzugte Substitutionen sind in der rechten Spalte dargestellt. Die Erfindung betrifft auch alle möglichen Kombinationen der gezeigten Aminosäure-Substitutionen:

**Tabelle 1:**

| AS-Posi-tion | Wildtyp AS | Substitutionen mit erhöhter Aktivität und Temperaturstabilität | bevorzugt | besonders bevorzugt |
|---|---|---|---|---|
| 9 | Leu | Ala, Arg, Asn, Asp, Cys, Gln, Glu, Gly, His, Ile, Lys, Met, Phe, Pro, Ser, Thr, Trp, Tyr, Val | Lys, Glu, Ile, Arg | Lys |
| 63 | Val | Ala, Arg, Asn, Asp, Cys, Gln, Glu, Gly, His, Ile, Leu, Lys, Met, Phe, Pro, Ser, Thr, Trp, Tyr | Ile, Met, Pro, Thr | Pro |
| 70 | Thr | Ala, Arg, Asn, Asp, Cys, Gin, Glu, Gly, His, Ile, Leu, Lys, Met, Phe, Pro, Ser, Trp, Tyr, Val | Ala, Cys, Asp, Glu, Phe, Gly, Ile, Leu, Met, Asn, Pro, Gln, Ser, Tyr, Arg, Val | Ile |
| 94 | Arg | Ala, Asn, Asp, Cys, Gln, Glu, Gly, His, Ile, Leu, Lys, Met, Phe, Pro, Ser, Thr, Trp, Tyr, Val | Glu, Phe, Gly, Lys, Met, Asn, Pro, Gln, Ala, Gly, His, Ile, Leu, Ser, Thr, Val, Tyr | Gln |
| 194 | Leu | Ala, Arg, Asn, Asp, Cys, Gln, Glu, Gly, His, Ile, Lys, Met. Phe, Pro, Ser, Thr, Trp, Tyr, Val | Cys, Lys, Met, Tyr | Tyr |
| 208 | Thr | Ala, Arg, Asn, Asp, Cys, Gln, Glu, Gly, His, Ile, Leu, Lys, Met, Phe, Pro, Ser, Trp, Tyr, Val | Ala, Gly, Leu | Ala |
| 250 | Cys | Ala, Arg, Asn, Asp, Gln, Glu, Gly, His, Ile, Leu, Lys, Met, Phe, Pro, Ser, Thr, Trp, Tyr, Val | Ala, Phe, Gly, Lys, Met, Arg, Ser, Thr, Tyr, His | Gly |
| 305 | Val | Ala, Arg, Asn, Asp, Cys, Gln, Glu, Gly, His, Ile, Leu, Lys, Met, Phe, Pro, Ser, Thr, Trp, Tyr | Leu, Met | Leu |
| 308 | Asp | Ala, Arg, Asn, Cys, Gln, Glu, Gly, His, Ile, Leu, Lys, Met, Phe, Pro, Ser, Thr, Trp, Tyr, Val | Asn, Trp | Asn |

Besonders bevorzugte Kombinationen beinhalten Enzyme die alle denkbaren Kombinationen der Substitutionen V63P, F168V und C250G enthalten, wie sie zum Beispiel bei den Enzym-Mutanten gemäß (Seq ID No: 3), (Seq ID No: 4), (Seq ID No: 5), und (Seq ID No: 6) gezeigt sind.

Die Erfindung betrifft somit Nitrilasen, bei denen im Vergleich zur Wildtyp Nitrilase aus Acidovorax facilis (Seq ID No: 2)
- der Rest Leu an der Position, welche Position 9 entspricht, ausgetauscht ist durch einen Rest ausgewählt aus der Gruppe bestehend aus Ala, Arg, Asn, Asp, Cys. Gln, Glu, Gly, His, Ile, Lys, Met, Phe, Pro, Ser, Thr, Trp, Tyr, Val; besonders bevorzugt ausgewählt aus der Gruppe bestehend aus Lys, Glu, Ile, Arg; insbesondere Lys; und/oder
- der Rest Val an der Position, welche Position 63 entspricht, ausgetauscht ist durch einen Rest ausgewählt aus der Gruppe bestehend aus Ala, Arg, Asn, Asp, Gys, Gln, Glu, Gly, His, Ile, Leu, Lys, Met, Phe, Pro, Ser, Thr, Trp, Tyr; besonders bevorzugt ausgewählt aus der Gruppe bestehend aus Pro, Ile, Met, Thr; insbesondere Pro; und/oder
- der Rest Thr an der Position, welche Position 70 entspricht, ausgetauscht ist durch einen Rest ausgewählt aus der Gruppe bestehend aus Ala, Arg, Asn, Asp, Cys, Gln, Glu, Gly, His, Ile, Leu, Lys, Met, Phe, Pro, Ser, Trp, Tyr, Val; besonders bevorzugt ausgewählt aus der Gruppe bestehend aus Ile, Ala, Cys, Asp, Glu, Phe, Gly, Leu, Met, Asn, Pro, Gin, Ser, Tyr, Arg, Val; insbesondere Ile; und/oder
- der Rest Arg an der Position, welche Position 94 entspricht, ausgetauscht ist durch einen Rest ausgewählt aus der Gruppe bestehend aus Ala, Asn, Asp, Cys, Gln, Glu, Gly, His, Ile, Leu, Cys, Met, Phe, Pro, Ser, Thr, Trp, Tyr, Val; besonders bevorzugt ausgewählt aus der Gruppe bestehend aus Gln, Glu, Phe, Gly, Lys, Met, Asn, Pro, Ala, Gly, His, Ile, Leu, Ser, Thr, Val, Tyr; insbesondere Gln und/oder
- der Rest Leu an der Position, welche Position 194 entspricht, ausgetauscht ist durch einen Rest ausgewählt aus der Gruppe bestehend aus Ala, Arg, Asn, Asp, Cys, Gln, Glu, Gly, His, Ile, Lys, Met, Phe, Pro, Ser, Thr, Trp, Tyr, Val; besonders bevorzugt ausgewählt aus der Gruppe bestehend aus Tyr, Cys, Lys, Met; insbesondere Tyr; und/oder
- der Rest Thr an der Position, welche Position 208 entspricht, ausgetauscht ist durch einen Rest ausgewählt aus der Gruppe bestehend aus Ala, Arg, Asn, Asp, Cys, Gln, Glu, Gly, His, Ile, Leu, Lys, Met, Phe, Pro, Ser, Trp, Tyr, Val; besonders bevorzugt ausgewählt aus der Gruppe bestehend aus Ala, Gly, Leu; insbesondere Ala; und/oder
- der Rest Cys an der Position, welche Position 250 entspricht, ausgetauscht ist durch einen Rest ausgewählt aus der Gruppe bestehend aus Ala, Arg, Asn, Asp, Gln, Glu, Gly, His, Ile, Leu, Lys, Met, Phe, Pro, Ser, Thr, Trp, Tyr, Val; besonders bevorzugt ausgewählt aus der Gruppe bestehend aus Gly, Ala, Phe, Lys, Met, Arg, Ser, Thr, Tyr, His; insbesondere Gly; und/oder
- der Rest Val an der Position, welche Position 305 entspricht, ausgetauscht ist durch einen Rest ausgewählt aus der Gruppe bestehend aus Ala, Arg, Asn, Asp, Cys, Gln, Glu, Gly, His, Ile, Leu, Lys, Met, Phe, Pro, Ser, Thr, Trp, Tyr; besonders bevorzugt ausgewählt aus der Gruppe bestehend aus Leu, Met; insbesondere Leu; und/oder
- der Rest Asp an der Position, welche Position 308 entspricht, ausgetauscht ist durch einen Rest ausgewählt aus der Gruppe bestehend aus Ala, Arg, Asn, Cys, Gln, Glu, Gly, His, Ile, Leu, Lys, Met, Phe, Pro, Ser, Thr, Trp, Tyr, Val; besonders bevorzugt ausgewählt aus der Gruppe bestehend aus Asn, Trp; insbesondere Asn.

Definiert man die Substitution an der Position, welche Position 9 entspricht, durch einen Aminosäurerest ausgewählt aus der Gruppe bestehend aus Lys, Glu, Ile, Arg; bevorzugt Lys; als "A"; die Substitution an der Position, welche Position 63 entspricht, durch einen Aminosäurerest ausgewählt aus der Gruppe bestehend aus Pro, Ile, Met, Thr; bevorzugt Pro; als "B"; die Substitution an der Position, welche Position 70 entspricht, durch einen Aminosäurerest ausgewählt aus der Gruppe bestehend aus Ile, Ala, Cys, Asp, Glu, Phe, Gly, Leu, Met, Asn, Pro, Gin, Ser, Tyr, Arg, Val; bevorzugt Ile; als "C"; die Substitution an der Position, welche Position 94 entspricht, durch einen Aminosäurerest ausgewählt aus der Gruppe bestehend aus Gln, Glu, Phe, Gly, Lys, Met, Asn, Pro, Ala, Gly, His, Ile, Leu, Ser, Thr, Val, Tyr; bevorzugt Gin; als "D"; die Substitution an der Position, welche Position 168 entspricht, durch Val als "E"; die Substitution an der Position, welche Position 194 entspricht, durch einen Aminosäurerest ausgewählt aus der Gruppe bestehend aus Tyr, Cys, Lys, Met; bevorzugt Tyr; als "F"; die Substitution an der Position, welche Position 208 entspricht, durch einen Aminosäurerest ausgewählt aus der Gruppe bestehend aus Ala, Gly, Leu; bevorzugt Ala; als "G"; die Substitution an der Position, welche Position 250 entspricht, durch einen Aminosäurerest ausgewählt aus der Gruppe bestehend aus Gly, Ala, Phe, Lys, Met, Arg, Ser, Thr, Tyr, His; bevorzugt Gly; als "H"; die Substitution an der Position, welche Position 305 entspricht, durch einen Aminosäurerest ausgewählt aus der Gruppe bestehend aus Leu, Met; bevorzugt Leu; als "I"; und die Substitution an der Position, welche Position 308 entspricht, durch einen Aminosäurerest ausgewählt aus der Gruppe bestehend aus Asn, Trp; bevorzugt Asn; als "J"; jeweils im Vergleich zur Wildtyp Nitrilase aus Acidovorax facilis (Seq ID No: 2), so lassen sich bevorzugte Ausführungsformen der erfindungsgemäßen Nitrilase in Kurzschreibweise wie folgt darstellen:
Einfache Substitutionen sind bevorzugt ausgewählt aus der Gruppe bestehend aus A, B, C, D, E, F, G, H, I und J. Dabei bedeutet beispielsweise der Buchstabe G, dass es sich um eine Nitrilase handelt, bei der im Vergleich zur Wildtyp Nitrilase aus Acidovorax facilis (Seq ID No: 2) an der Position, welche Position 208 entspricht Thr des Wildtyps durch Ala, Gly oder Leu ausgetauscht wurde, ansonsten jedoch alle Aminosäurepositionen dem Wildtyp entsprechen.

Zweifache Substitutionen sind in entsprechender Schreibweise bevorzugt ausgewählt aus der Gruppe bestehend aus AB, AC, AD, AE, AF, AG, AH, AI, AJ, BC, BD, BE, BF, BG, BH, BI, BJ, CD, CE, CF, CG, CH, CI, CJ, DE, DF, DG, DH, DI, DJ, EF, EG, EH, EI, EJ, FG, FH, FI, FJ, GH, GI, GJ, HI, HJ und IJ.

Dreifache Substitutionen sind in entsprechender Schreibweise bevorzugt ausgewählt aus der Gruppe bestehend aus ABC, ABD, ABE, ABF, ABG, ABH, ABI, ABJ, ACD, ACE, ACF, ACG, ACH, ACI, ACJ, ADE, ADF, ADG, ADH, ADI, ADJ, AEF, AEG, AEH, AEI, AEJ, AFG, AFH, AFI, AFJ, AGH, AGI, AGJ, AHI, AHJ, AIJ, BCD, BCE, BCF, BCG, BCH, BCI, BCJ, BDE, BDF, BDG, BDH, BDI, BDJ, BEF, BEG, BEH, BEI, BEJ, BFG, BFH, BFI, BFJ, BGH, BGI, BGJ, BHI, BHJ, BIJ, CDE, CDF, CDG, CDH, CDI, CDJ, CEF, CEG, CEH, CEI, CEJ, CFG, CFH, CFI, CFJ, CGH, CGI, CGJ, CHI, CHJ, CIJ, DEF, DEG, DEH, DEI, DEJ, DFG, DFH, DFI, DFJ, DGH, DGI, DGJ, DHI, DHJ, DIJ, EFG, EFH, EFI, EFJ, EGH, EGI, EGJ, EHI, EHJ, EIJ, FGH, FGI, FGJ, FHI, FHJ, FIJ, GHI, GHJ, GIJ, und HIJ.

In einer bevorzugten Ausführungsform weist die erfindungsgemäße Nitrilase im Vergleich zur Wildtyp Nitrilase aus Acidovorax facilis (Seq ID No: 2) wenigstens vier Substitutionen an den Positionen, welche Positionen 9, 63, 70, 94, 168, 194, 208, 250, 305, oder 308 entsprechen, auf, wobei drei dieser Substitutionen ausgewählt sind aus der Gruppe bestehend aus ABC, ABD, ABE, ABF, ABG, ABH, ABI, ABJ, ACD, ACE, ACF, ACG, ACH, ACI, ACJ, ADE, ADF, ADG, ADH, ADI, ADJ, AEF, AEG, AEH, AEI, AEJ, AFG, AFH, AFI, AFJ, AGH, AGI, AGJ, AHI, AHJ, AIJ, BCD, BCE, BCF, BCG, BCH, BCI, BCJ, BDE, BDF, BDG, BDH, BDI, BDJ, BEF, BEG, BEH, BEI, BEJ, BFG, BFH, BFI, BFJ, BGH, BGI, BGJ, BHI, BHJ, BIJ, CDE, CDF, CDG, CDH, CDI, CDJ, CEF, CEG, CEH, CEI, CEJ, CFG, CFH, CFI, CFJ, CGH, CGI, CGJ, CHI, CHJ, CIJ, DEF, DEG, DEH, DEI, DEJ, DFG, DFH, DFI, DFJ, DGH, DGI, DGJ, DHI, DHJ, DIJ, EFG, EFH, EFI, EFJ, EGH, EGI, EGJ, EHI, EHJ, EIJ, FGH, FGI, FGJ, FHI, FHJ, FIJ, GHI, GHJ, GIJ, und HIJ.

In einer bevorzugten Ausführungsform weist die erfindungsgemäße Nitrilase im Vergleich zur Wildtyp Nitrilase aus Acidovorax facilis (Seq ID No: 2) wenigstens fünf Substitutionen an Positionen, welche Positionen 9, 63, 70, 94, 168, 194, 208, 250, 305, oder 308 entsprechen, auf, wobei drei dieser Substitutionen ausgewählt sind aus der Gruppe bestehend aus ABC, ABD, ABE, ABF, ABG, ABH, ABI, ABJ, ACD, ACE, ACF, ACG, ACH, ACI, ACJ, ADE, ADF, ADG, ADH, ADI, ADJ, AEF, AEG, AEH, AEI, AEJ, AFG, AFH, AFI, AFJ, AGH, AGI, AGJ, AHI, AHJ, AIJ, BCD, BCE, BCF, BCG, BCH, BCI, BCJ, BDE, BDF, BDG, BDH, BDI, BDJ, BEF, BEG, BEH, BEI, BEJ, BFG, BFH, BFI, BFJ, BGH, BGI, BGJ, BHI, BHJ, BIJ, CDE, CDF, CDG, CDH, CDI, CDJ, CEF, CEG, CEH, CEI, CEJ, CFG, CFH, CFI, CFJ, CGH, CGI, CGJ, CHI, CHJ, CIJ, DEF, DEG, DEH, DEI, DEJ, DFG, DFH, DFI, DFJ, DGH, DGI, DGJ, DHI, DHJ, DIJ, EFG, EFH, EFI, EFJ, EGH, EGI, EGJ, EHI, EHJ, EIJ, FGH, FGI, FGJ, FHI, FHJ, FIJ, GHI, GHJ, GIJ, und HIJ.

In einer bevorzugten Ausführungsform weist die erfindungsgemäße Nitrilase im Vergleich zur Wildtyp Nitrilase aus Acidovorax facilis (Seq ID No: 2) wenigstens sechs Substitutionen an Positionen, welche Positionen 9, 63, 70, 94, 168, 194, 208, 250, 305, oder 308 entsprechen, auf, wobei drei dieser Substitutionen ausgewählt sind aus der Gruppe bestehend aus ABC, ABD, ABE, ABF, ABG, ABH, ABI, ABJ, ACD, ACE, ACF, ACG, ACH, ACI, ACJ, ADE, ADF, ADG, ADH, ADI, ADJ, AEF, AEG, AEH, AEI, AEJ, AFG, AFH, AFI, AFJ, AGH, AGI, AGJ, AHI, AHJ, AIJ, BCD, BCE, BCF, BCG, BCH, BCI, BCJ, BDE, BDF, BDG, BDH, BDI, BDJ, BEF, BEG, BEH, BEI, BEJ, BFG, BFH, BFI, BFJ, BGH, BGI, BGJ, BHI, BHJ, BIJ, CDE, CDF, CDG, CDH, CDI, CDJ, CEF, CEG, CEH, CEI, CEJ, CFG, CFH, CFI, CFJ, CGH, CGI, CGJ, CHI, CHJ, CIJ, DEF, DEG, DEH, DEI, DEJ, DFG, DFH, DFI, DFJ, DGH, DGI, DGJ, DHI, DHJ, DIJ, EFG, EFH, EFI, EFJ, EGH, EGI, EGJ, EHI, EHJ, EIJ, FGH, FGI, FGJ, FHI, FHJ, FIJ, GHI, GHJ, GIJ, und HIJ.

In einer bevorzugten Ausführungsform weist die erfindungsgemäße Nitrilase im Vergleich zur Wildtyp Nitrilase aus Acidovorax facilis (Seq ID No: 2) wenigstens sieben Substitutionen an Positionen, welche Positionen 9, 63, 70, 94, 168, 194, 208, 250, 305, oder 308 entsprechen, auf, wobei drei dieser Substitutionen ausgewählt sind aus der Gruppe bestehend aus ABC, ABD, ABE, ABF, ABG, ABH, ABI, ABJ, ACD, ACE, ACF, ACG, ACH, ACI, ACJ, ADE, ADF, ADG, ADH, ADI, ADJ, AEF, AEG, AEH, AEI, AEJ, AFG, AFH, AFI, AFJ, AGH, AGI, AGJ, AHI, AHJ, AIJ, BCD, BCE, BCF, BCG, BCH, BCI, BCJ, BDE, BDF, BDG, BDH, BDI, BDJ, BEF, BEG, BEH, BEI, BEJ, BFG, BFH, BFI, BFJ, BGH, BGI, BGJ, BHI, BHJ, BIJ, CDE, CDF, CDG, CDH, CDI, CDJ, CEF, CEG, CEH, CEI, CEJ, CFG, CFH, CFI, CFJ, CGH, CGI, CGJ, CHI, CHJ, CIJ, DEF, DEG, DEH, DEI, DEJ, DFG, DFH, DFI, DFJ, DGH, DGI, DGJ, DHI, DHJ, DIJ, EFG, EFH, EFI, EFJ, EGH, EGI, EGJ, EHI, EHJ, EIJ, FGH, FGI, FGJ, FHI, FHJ, FIJ, GHI, GHJ, GIJ, und HIJ.

In einer besonders bevorzugten Ausführungsform weist die erfindungsgemäße Nitrilase wenisgtens drei, vier, fünf, sechs, sieben, acht, neun oder zehn Substitutionen im Vergleich zur Wildtyp Nitrilase aus Acidovorax facilis (Seq ID No: 2) auf, umfassend die drei Substitutionen V63P, F168V und C250G.

Eine weitere erfindungsgemäße Nitrilase wurde aus einem unkultivierbaren Organismus gewonnen und ist in (Seq ID No: 7) gezeigt. Durch eine geeignete Kombination der Sequenzen aus (Seq ID No: 1) mit (Seq ID No: 7) und Einfügen einer Kombination der in Tabelle 1 gezeigten Substitutionen wurde ein Enzym der (Seq ID No: 8) erzeugt, das überraschenderweise eine stark gesteigerte Aktivität und Temperaturstabilität im Vergleich mit dem Ausgangsenzym aufweist.

Die erfindungsgemäße Nitrilase weist bevorzugt im Vergleich zu der Aminosäuresequenz der Nitrilase aus *Acidovorax facilis* (Seq ID No: 2) eine Homologie von mindestens 60% oder mindestens 65%, bevorzugt mindestens 70% oder mindestens 75%, besonders bevorzugt mindestens 80% oder mindestens 85%, ganz besonders bevorzugt mindestens 90% oder mindestens 92%, ebenso bevorzugt mindestens 93% oder mindestens 94%, sowie besonders bevorzugt mindestens 95%, mindestens 96%, mindestens 97% oder mindestens 98% und am bevorzugtesten mindestens 99% auf.

Im Sinne der Erfindung wird die Homologie einer Sequenz berechnet als Identität mittels BLASTP 2.2.20+ (Stephen F. Altschul, Thomas L. Madden, Alejandro A. Schäffer, Jinghui Zhang, Zheng Zhang, Webb Miller, and David J. Lipman (1997)). "Gapped BLAST and PSI-BLAST: a new generation of protein database search programs", Nucleic Acids Res. 25:33899-3402; Stephen F. Altschul, John C. Wootton, E. Michael Gertz, Richa Agarwala, Aleksandr Morgulis, Alejandro A. Schäffer, and Yi-Kuo Yu (2005)".

Die Position der beanspruchten Substitutionen wird bei zu der Aminosäuresequenz der Nitrilase aus *Acidovorax facilis* (Seq ID No: 2) homologen Nitrilasen aus einem Sequenz- oder Struktur-basierten Alignment bestimmt. Methoden und Werkzeuge für solche Alignments sind dem Fachmann bekannt. So werden für Sequenz-basierte Aligmnents zum Beispiel ClustalW (Larkin M.A., Blackshields G., Brown N.P., Chenna R., McGettigan P.A., McWilliam H., Valentin F., Wallace I.M., Wilm A.. Lopez R., Thompson J.D., Gibson T.J. and Higgins D.G. (2007), Bioinformatics 23(21): 2947-2948), Multialign (F. CORPET, 1988, Nucl. Acids Res., 16 (22), 10881-10890) oder Dialign (Subramanian AR, Hiran S, Steinkamp R, Meinicke P, Corel E, Morgenstern B., Nucleic Acids Res. 2010 Jul 1;38 Suppl:W19-22) und Struktur-basierte Alignments durch Swiss-Model (Arnold K., Bordoli L., Kopp J., and Schwede T. (2006), Bioinformatics, 22.195-201.), CPHmodels (Nielsen M., Lundegaard C., Lund O., Petersen TN, Nucleic Acids Research, 2010, Vol. 38) oder Geno3D (Combet C, Jambon M, Deléage G & Geourjon C, Bioinformatics, 2002, 18, 213-214) genutzt.

Die entsprechenden Positionen in den homologen Sequenzen können somit durch Insertion oder Deletion von einer oder mehreren Aminosäuren enstprechend verschoben sein. Weiterhin ist es ebenso möglich, dass die entsprechend homologen Nitrilasen an der durch die Aligments zugeordneten Positionen eine andere Aminosäure als die Nitrilase aus *Acidovorax facilis* (Seq ID No: 2) aufweisen, so dass zum Beispiel Leu9 aus Seq ID No. 2 in der homologen Nitrilase 1 Ile11, in der homologen Nitrilase 2 Val8 oder in der homologen Nitrilase X Aminosäure Y entspricht. Für alle anderen genannten Positionen gilt dies entsprechend.

Das erfindungsgemäße Enzym entspricht bevorzugt einer Nitrilase mit mehr als 60% Homologie zu der Aminosäuresequenz gemäß (Seq ID No: 2), welche mindestens 1, bevorzugt mindestens 2, bevorzugter mindestens 3, besonders bevorzugt mindestens 4, ebenso bevorzugt mindestens 5 und am bevorzugtesten mindestens 6 Aminosäure-Substitution(en) ausgewählt aus der folgenden Gruppe aufweist:
a) eine Substitution in der Position, die Position 9 in (Seq ID No: 2) entspricht
b) eine Substitution in der Position, die Position 63 in (Seq ID No: 2) entspricht
c) eine Substitution in der Position, die Position 70 in (Seq ID No: 2) entspricht
d) eine Substitution in der Position, die Position 94 in (Seq ID No: 2) entspricht
e) eine Substitution in der Position, die Position 194 in (Seq ID No: 2) entspricht
f) eine Substitution in der Position, die Position 208 in (Seq ID No: 2) entspricht
g) eine Substitution in der Position, die Position 250 in (Seq ID No: 2) entspricht
h) eine Substitution in der Position, die Position 305 in (Seq ID No: 2) entspricht
i) eine Substitution in der Position, die Position 308 in (Seq ID No: 2) entspricht
und/oder das Enzym im Vergleich zum Ausgangsenzym eine um mindestens 15% gesteigerte Aktivität bei der Umsetzung eines Nitrils zu einer Carbonsäure aufweist und/oder das Enzym nach einer Temperaturbehandlung, bei der die Aktivität des Ausgangsenzym mindestens um 40% reduziert wird, im Vergleich zum Ausgangsenzym eine um mindestens 10% erhöhte Restaktivität besitzt.

Bevorzugt ist das erfindungsgemäße Enzym, welches zusätzlich zu den oben genannten Substitutionen noch mindestens 1, bevorzugt mindestens 2, bevorzugter mindestens 3, besonders bevorzugt mindestens 4 und am bevorzugtesten mindestens 5 zusätzliche Aminosäure-Substitution(en) ausgewählt aus der folgenden Gruppe aufweist:
a) der Rest Tyr an der Position, welche Position 65 in (Seq ID No. 2) entspricht, ausgetauscht ist durch einen Rest ausgewählt aus der Gruppe bestehend aus Ala, Arg, Asn, Asp, Cys, Gln, Glu, Gly, His, Ile, Leu, Lys, Met, Phe, Pro, Ser, Thr, Trp, Val; besonders bevorzugt Cys;
b) der Rest Phe an der Position, welche Position 168 in (Seq ID No. 2) entspricht, ausgetauscht ist durch einen Rest ausgewählt aus der Gruppe bestehend aus Ala, Arg, Asn, Asp, Cys, Gln, Glu, Gly, His, Ile, Leu, Lys, Met, Pro, Ser, Thr, Trp, Tyr, Val; besonders bevorzugt Val.
c) der Rest Phe an der Position, welche Position 174 In (Seq ID No. 2) entspricht, ausgetauscht ist durch einen Rest ausgewählt aus der Gruppe bestehend aus Ala, Arg, Asn, Asp, Cys, Gln, Glu, Gly, His, Ile, Leu, Lys, Met, Pro, Ser, Thr, Trp, Tyr, Val; besonders bevorzugt Ile;
d) der Rest Leu an der Position, welche Position 201 in (Seq ID No. 2) entspricht, ausgetauscht ist durch einen Rest ausgewählt aus der Gruppe bestehend aus Ala, Arg, Asn, Asp, Cys, Gln, Glu, Gly, His, Ile, Lys, Met, Phe, Pro, Ser, Thr, Trp, Tyr, Val; besonders bevorzugt Asn;
e) der Rest Thr an der Position, welche Position 210 in (Seq ID No. 2) entspricht, ausgetauscht ist durch einen Rest ausgewählt aus der Gruppe bestehend aus Ala, Arg, Asn, Asp, Cys, Gln, Glu, Gly, His, Ile, Leu, Lys, Met, Phe, Pro, Ser, Trp, Tyr, Val; besonders bevorzugt Ala.

In einer bevorzugten Ausführungsform weist die erfindungsgemäße Nitrilase eine der folgenden Mutations-Kombinationen auf:
a) V63P, C250G (Seq ID No: 3)
b) F168V, C250G (Seq ID No: 4).

In einer bevorzugten Ausführungsform ist die erfindungsgemäße Nitrilase die Nitrilase gemäß (Seq ID No: 8) oder eine homologe Nitrilase, welche die gleichen Mutationen zu ihrem Ausgangsenzym, wie (Seq ID No: 8) im Vergleich zu (Seq ID No: 2) aufweist. Die Nitrilase gemäß (Seq ID No: 8) weist im Vergleich zum Ausgangsenzym mit CH-Dinitril eine um 335% erhöhte Aktivität auf.

Bevorzugt weist die erfindungsgemäße Nitrilase eine Identität zur (Seq ID No: 8) von mehr als 90%, bevorzugt mehr als 93%, besonders bevorzugt mehr als 95%, ganz besonders bevorzugt mehr als 98% und am bevorzugtesten von mehr als 99% auf.

Die erfindungsgemäße Nitrilase weist im Vergleich zum Ausgangsenzym (Seq ID No: 2) bevorzugt eine um 20%, bevorzugt um 30%, bevorzugter um 40%, besonders bevorzugt um 60%, ebenfalls bevorzugt um 80%, noch bevorzugter um 150% and am bevorzugtesten um 200% gesteigerte Aktivität bei der Umsetzung eines Nitrils zu einer Carbonsäure auf.

In bevorzugten Ausführungsformen A1-A5 bis F1-F5 weist die erfindungsgemäße Nitrilase nach einer Temperatur-Behandlung, bei der die Aktivität des Ausgangsenzym mindestens um die in der nachstehenden Tabelle angegebene Prozentsätze reduziert wird, im Vergleich mit dem Ausgangsenzym (Seq ID No: 2) mindestens die in der nachstehenden Tabelle 2 angegebenen Rest-Aktivitäten auf:

**Tabelle 2:**

| Nr. | Inaktivierung Ausgangsenzym | Erhöhte Restaktivität der beanspruchten Nitrilase | | | | | |
|---|---|---|---|---|---|---|---|
| | mindestens | A | B | C | D | E | F |
| 1 | 20% | 10% | 20% | 30% | 50% | 100% | 200% |
| 2 | 40% | 10% | 20% | 30% | 50% | 100% | 200% |
| 3 | 60% | 10% | 20% | 30% | 50% | 100% | 200% |
| 4 | 80% | 10% | 20% | 30% | 50% | 100% | 200% |
| 5 | 90% | 10% | 20% | 30% | 50% | 100% | 200% |

In vorstehender Tabelle bedeutet beispielsweise Ausführungsform C3, dass die efindungsgemäße Nitrilase nach einer Temperatur-Behandlung, bei der die Aktivität des Ausgangsenzym mindestens um 60% reduziert wird, im Vergleich mit dem Ausgangsenzym eine um wenigstens 30%, bevorzugt um wenigstens 50%, noch bevorzugter um wenigstens 100% und am bevorzugtesten um wenigstens 200% erhöhte Restaktivität aufweist.

Ein weiterer Aspekt der Erfindung betrifft ein Verfahren zur Herstellung einer Carbonsäure aus Nitrilen durch in Kontaktbringen eines Nitrils mit einer Nitrilase gemäß einem der vorstehenden Ansprüche. Vorzugsweise erfolgt die Umsetzung in Gegenwart von Wasser, d.h. in einem wässrigen Medium. Bevorzugt dient das Verfahren der Herstellung einer Carbonsäure ausgewählt aus der Gruppe bestehend aus 4-Cyano-Pentansäure 2-(1-Cyanocyclohexyl)essigsäure, sowie Benzoesäure.

Die nachfolgenden Beispiele dienen der näheren Erläuterung der Erfindung, sind jedoch nicht einschränkend auszulegen.

### Beispiele

Die Enzymvarianten wurden mit Hilfe herkömmlicher Verfahren hergestellt und im Hinblick auf ihre Eigenschaften untersucht. Für die Bereitstellung der Enzymvarianten wurden zunächst die Mutationen auf Gen-Ebene mittels üblicher molekularbiologischer Methoden eingeführt. Die Gene der Enzym-Varianten wurden in Expressionsvektoren kloniert. Mit diesem wurden dann *Escherichia coli* Expressions-Stämme transformiert. Das DNA Plasmid beinhaltete die Information für die Expressions-Regulation der Enzymvarianten.. Die kodierende Sequenz des Enzyms oder der Enzymvariante wurde dabei unter die Kontrolle eines induzierbaren Promoters gestellt. Dadurch ließ sich durch Zugabe eines Induktors die Expression der Enzymvarianten steuern (i.d.R. wurde Isopropyl-β-D-thiogalactopyranosid (IPTG) verwendet). Die so transformierten *E. coli* Stämme wurden dann in herkömmlichen Nährmedien (z.B. Lennox-Broth, Minimalmedium M9) kultiviert und mit IPTG induziert. Nach Expression wurde die Biomasse durch Zentrifugation geerntet. Aus der Biomasse wurden dann die Enzymvarianten nach entprechender Zelllyse gewonnen und gereinigt. Dabei wurden Zentrifugations-, Fällungs-, Ultrafiltrations- und/oder Chromatographieverfahren eingesetzt.

Die beschriebenen Enzymvarianten weisen die in Tabelle 3 gezeigten Aktivitätssteigerungen im Vergleich zum Ausgangsenzym mit 3 unterschiedlichen Nitrilsubstraten auf.

**Tabelle 3:**

| Pos. | Enzym | 2MG | CH-Dinitril | Benzonitril |
|---|---|---|---|---|
| | (Seq ID No: 2) | 100% | 100% | 100% |
| 9 | Leu9Lys | 134% | 131% | 98% |
| | Leu9glu | 160% | 177% | 114% |
| | Leu9Ile | 147% | 171% | 100% |
| 63 | Val63Ile | 163% | 238% | 90% |
| | Val63Met | 165% | 183% | 97% |
| | Val63Pro | 142% | 198% | 89% |
| | Val63Thr | 173% | 175% | 99% |
| 70 | Thr70Ala | 124% | 183% | 111% |
| | Thr70Cys | 149% | 153% | 128% |
| | Thr70Asp | 136% | 224% | 114% |
| | Thr70Glu | 125% | 207% | 113% |
| | Thr70Phe | 132% | 170% | 104% |
| | Thr70Gly | 134% | 230% | 114% |
| | Thr70Ile | 123% | 151% | 113% |
| | Thr70Leu | 128% | 200% | 105% |
| | Thr70Met | 122% | 180% | 102% |
| | Thr70Asn | 122% | 216% | 108% |
| | Thr70Pro | 126% | 206% | 99% |
| | Thr70Gln | 129% | 222% | 118% |
| | Thr70Ser | 138% | 177% | 122% |
| | Thr70Tyr | 130% | 164% | 103% |
| 94 | Arg94Glu | 111% | 107% | 97% |
| | Arq94Phe | 117% | 97% | 113% |
| | Arg94Gly | 123% | 63% | 110% |
| | Arg94Lys | 112% | 98% | 95% |
| | Arg94Met | 125% | 80% | 110% |
| | Arg94Asn | 120% | 81% | 101% |
| | Arg94Pro | 113% | 97% | 101% |
| | Arg94Gln | 118% | 101% | 108% |
| 194 | Leu194Cys | 117% | 105% | 71% |
| | Leu194Lys | 121% | 67% | 73% |
| | Leu194Met | 116% | 121% | 68% |
| | Leu194Tyr | 118% | 122% | 76% |
| 208 | Thr208Ala | 144% | 194% | 124% |
| | Thr208Gly | 154% | 181% | 131% |
| | Thr208Leu | 132% | 152% | 123% |
| 250 | Cys250Ala | 112% | 221% | 41% |
| | Cys250Phe | 111% | 194% | 40% |
| | Cys250Gly | 126% | 281% | 40% |
| | Cys250Lys | 135% | 97% | 45% |
| | Cys250Met | 125% | 146% | 40% |
| | Cys250Arg | 124% | 165% | 39% |
| | Cys250Ser | 128% | 246% | 40% |
| | Cys250Thr | 104% | 186% | 38% |
| | Cys250Tyr | 116% | 196% | 39% |
| 305 | Val305Leu | 126% | 98% | 116% |
| | Val305Met | 128% | 76% | 116% |
| 308 | Asp308Asn | 133% | 118% | 104% |
| | Asp308Trp | 128% | 105% | 92% |

Tabelle 4 zeigt erfindungsgemäße Einzelsubstitutionen und deren Restaktivitäten nach einer 15 minütigen Behandlung bei 55°C oder 60°C.

**Tabelle 4:**

| Pos. | Enzym | 55°C | Erhöhung der RestAktivität in % | 60°C | Erhöhung der RestAktivität in % |
|---|---|---|---|---|---|
| | Seq ID No: 2 | 57% | | 14% | |
| 9 | Leu9Lys | 80% | 40% | 20% | 43% |
| | Leu9Glu | 83% | 45% | 35% | 151% |
| | Leu9Ile | 73% | 28% | 46% | 229% |
| | Leu9Arg | 89% | 56% | 71% | 409% |
| 63 | Val63Pro | 68% | 19% | 19% | 36% |
| | Val63Ile | 64% | 12% | 15% | 7% |
| 70 | Thr70Ile | 68% | 19% | 25% | 79% |
| | Thr70Cys | 59% | 4% | 30% | 118% |
| | Thr70Phe | 76% | 33% | 4% | -71% |
| | Thr70Leu | 85% | 50% | 22% | 56% |
| | Thr70Met | 67% | 18% | 19% | 37% |
| | Thr70Arq | 82% | 44% | 40% | 188% |
| | Thr70Val | 91% | 60% | 41% | 190% |
| | Thr70Tyr | 98% | 72% | 46% | 231% |
| 94 | Arg94Gln | 81% | 42% | 18% | 29% |
| | Arg94Ala | 90% | 58% | 66% | 370% |
| | Arg94Glu | 82% | 44% | 38% | 173% |
| | Arg94Phe | 85% | 49% | 16% | 16% |
| | Arg94Gly | 89% | 57% | 8% | -40% |
| | Arg94His | 79% | 39% | 30% | 112% |
| | Arg94Ile | 96% | 68% | 63% | 354% |
| | Arg94Lys | 85% | 49% | 49% | 247% |
| | Arg94Leu | 96% | 69% | 68% | 385% |
| | Arg94Met | 93% | 63% | 6% | -56% |
| | Arg94Asn | 91% | 60% | 40% | 189% |
| | Arg94Pro | 77% | 35% | 36% | 156% |
| | Arg94Ser | 93% | 64% | 57% | 310% |
| | Arg94Thr | 88% | 54% | 42% | 198% |
| | Arg94Val | 77% | 35% | 29% | 111% |
| | Arg94Tyr | 95% | 66% | 59% | 320% |
| 168 | Phe168Val | 94% | 65% | 62% | 343% |
| 250 | Cys250Gly | 80% | 40% | 20% | 43% |
| | Cys250Ala | 85% | 49% | 40% | 183% |
| | Cys250Phe | 90% | 58% | 48% | 240% |
| | Cys250His | 98% | 73% | 61% | 335% |
| | Cys250Met | 79% | 38% | 44% | 213% |
| | Cys250Arg | 77% | 35% | 36% | 158% |
| | Cys250Ser | 61% | 7% | 21% | 51% |
| | Cys250Tyr | 99% | 73% | 68% | 384% |
| 305 | Val305Leu | 99% | 74% | 28% | 100% |
| | Val305Met | 79% | 39% | 45% | 219% |

Tabelle 5 zeigt die Aktivität von erfindungsgemäßen Mehrfachmutanten im Vergleich zum Ausgangsenzym (Seq ID No: 2), bei denen die Kombination von Einzelsubstitutionen zu einer synergistischen Verbesserung der Aktivität des Enzyms führt:

**Tabelle 5:**

| | Substrat | |
|---|---|---|
| Enzym-Mutante | 2MG | Benzonitril |
| Seq ID No: 2 | 100% | 100% |
| V63P | 142% | 89% |
| C250G | 126% | |
| V63P / F168V (Seq ID No: 5) | | 171% |
| V63P / C250G (Seq ID No: 3) | 206% | |

## Patentansprüche

1. Nitrilase umfassend eine Aminosäuresequenz mit mindestens 60% Identität zur Aminosäuresequenz gemäß Seq ID No: 2, welche im Vergleich zur Nitrilase gemäß Seq ID No: 2
(i) eine um mindestens 15% gesteigerte Aktivität bei der Umsetzung eines Nitrils zur entsprechenden Carbonsäure aufweist, wobei das Nitril ausgewählt ist aus der Gruppe bestehend aus 2-Methylglutaronitril, 1-(Cyanomethyl)cyclohexan-1-carbonitril und Benzonitril;
und
(ii) mindestens eine Aminosäure-Substitution aufweist ausgewählt aus der Gruppe bestehend aus:
a) Substitution von Leu in der Position, welche Position 9 von Seq ID No: 2 entspricht, durch eine Aminosäure ausgewählt aus der Gruppe bestehend aus Ala, Arg, Asn, Asp, Cys, Gin, Glu, Gly, His, Ile, Lys, Met, Phe, Pro, Ser, Thr, Trp, Tyr und Val;
b) Substitution von Val in der Position, welche Position 63 von Seq ID No: 2 entspricht, durch eine Aminosäure ausgewählt aus der Gruppe bestehend aus Ala, Arg, Asn, Asp, Cys, Gln, Glu, Gly, His, Ile, Leu, Lys, Met, Phe, Pro, Ser, Thr, Trp und Tyr;
c) Substitution von Thr in der Position, welche Position 70 von Seq ID No: 2 entspricht, durch eine Aminosäure ausgewählt aus der Gruppe bestehend aus Ala, Arg, Asn, Asp, Cys, Gin, Glu, Gly, His, Ile, Leu, Lys, Met, Phe, Pro, Ser, Trp, Tyr und Val;
d) Substitution von Arg in der Position, welche Position 94 von Seq ID No: 2 entspricht, durch eine Aminosäure ausgewählt aus der Gruppe bestehend aus Ala, Asn, Asp, Cys, Gin, Glu, Gly, His, Ile, Leu, Lys, Met, Phe, Pro, Ser, Thr, Trp, Tyr und Val;
e) Substitution von Leu in der Position, welche Position 194 von Seq ID No: 2 entspricht, durch eine Aminosäure ausgewählt aus der Gruppe bestehend aus Ala, Arg, Asn, Asp, Cys, Gin, Glu, Gly, His, Ile, Lys, Met, Phe, Pro, Ser, Thr, Trp, Tyr und Val;
f) Substitution von Thr in der Position, welche Position 208 von Seq ID No: 2 entspricht, durch eine Aminosäure ausgewählt aus der Gruppe bestehend aus Ala, Arg, Asn, Asp, Cys, Gin, Glu, Gly, His, Ile, Leu, Lys, Met, Phe, Pro, Ser, Trp, Tyr und Val;
g) Substitution von Cys in der Position, welche Position 250 von Seq ID No: 2 entspricht, durch eine Aminosäure ausgewählt aus der Gruppe bestehend aus Ala, Arg, Asn, Asp, Gln, Glu, Gly, His, Ile, Leu, Lys, Met, Phe, Pro, Ser, Thr, Trp, Tyr und Val;
h) Substitution von Val in der Position, welche Position 305 von Seq ID No: 2 entspricht, durch eine Aminosäure ausgewählt aus der Gruppe bestehend aus Ala, Arg, Asn, Asp, Cys, Gin, Glu, Gly, His, Ile, Leu, Lys, Met, Phe, Pro, Ser, Thr, Trp und Tyr; und
i) Substitution von Asp in der Position, welche Position 308 von Seq ID No: 2 entspricht, durch eine Aminosäure ausgewählt aus der Gruppe bestehend aus Ala, Arg, Asn, Cys, Gin, Glu, Gly, His, Ile, Leu, Lys, Met, Phe, Pro, Ser, Thr, Trp, Tyr und Val.

2. Nitrilase nach Anspruch 1, welche mindestens eine Aminosäure-Substitution aufweist ausgewählt aus der Gruppe bestehend aus:
a) Substitution von Leu in der Position, welche Position 9 von Seq ID No: 2 entspricht, durch Lys, Glu, Ile oder Arg;
b) Substitution von Val in der Position, welche Position 63 von Seq ID No: 2 entspricht, durch Pro, Ile, Met oder Thr;
c) Substitution von Thr in der Position, welche Position 70 von Seq ID No: 2 entspricht, durch Ile, Ala, Cys, Asp, Glu, Phe, Gly, Leu, Met, Asn, Pro, Gin, Ser, Tyr; Arg oder Val;
d) Substitution von Arg in der Position, welche Position 94 von Seq ID No: 2 entspricht, durch Gin, Glu, Phe, Gly, Lys, Met, Asn, Pro, Ala, Gly, His, Ile, Leu, Ser, Thr, Val oder Tyr;
e) Substitution von Leu in der Position, welche Position 194 von Seq ID No: 2 entspricht, durch Tyr, Cys, Lys oder Met;
f) Substitution von Thr in der Position, welche Position 208 von Seq ID No: 2 entspricht, durch Ala, Gly oder Leu;
g) Substitution von Cys in der Position, welche Position 250 von Seq ID No: 2 entspricht, durch Gly, Ala, Phe, Lys, Met, Arg, Ser, Thr, Tyr oder His;
h) Substitution von Val in der Position, welche Position 305 von Seq ID No: 2 entspricht, durch Leu oder Met; und
i) Substitution von Asp in der Position, welche Position 308 von Seq ID No: 2 entspricht, durch Asn oder Trp.

3. Nitrilase nach Anspruch 1 oder 2, welche mindestens zwei Aminosäure-Substitutionen aufweist ausgewählt aus der Gruppe bestehend aus a), b), c), d), e), f), g), h) und i).

4. Nitrilase nach einem der vorstehenden Ansprüche, welche mindestens drei Aminosäure-Substitutionen aufweist ausgewählt aus der Gruppe bestehend aus a), b), c), d), e), f), g), h) und i).

5. Nitrilase nach einem der vorstehenden Ansprüche, welche zusätzlich mindestens eine weitere Aminosäure-Substitution aufweist ausgewählt aus der Gruppe bestehend aus:
j) Substitution von Tyr in der Position, welche Position 65 von Seq ID No: 2 entspricht, durch eine Aminosäure ausgewählt aus der Gruppe bestehend aus Ala, Arg, Asn, Asp, Cys, Gln, Glu, Gly, His, Ile, Leu, Lys, Met, Phe, Pro, Ser, Thr, Trp und Val;
k) Substitution von Phe in der Position, welche Position 168 von Seq ID No: 2 entspricht, durch eine Aminosäure ausgewählt aus der Gruppe bestehend aus Ala, Arg, Asn, Asp, Cys, Gin, Glu, Gly, His, Ile, Leu, Lys, Met, Pro, Ser, Thr, Trp, Tyr und Val;
l) Substitution von Phe in der Position, welche Position 174 von Seq ID No: 2 entspricht, durch eine Aminosäure ausgewählt aus der Gruppe bestehend aus Ala, Arg, Asn, Asp, Cys, Gln, Glu, Gly, His, Ile, Leu, Lys, Met, Pro, Ser, Thr, Trp, Tyr und Val;
m) Substitution von Ile in der Position, welche Position 201 von Seq ID No: 2 entspricht, durch eine Aminosäure ausgewählt aus der Gruppe bestehend aus Ala, Arg, Asn, Asp, Cys, Gin, Glu, Gly, His, Leu, Lys, Met, Phe, Pro, Ser, Thr, Trp, Tyr und Val; und
n) Substitution von Thr in der Position, welche Position 210 von Seq ID No: 2 entspricht, durch eine Aminosäure ausgewählt aus der Gruppe bestehend aus Ala, Arg, Asn, Asp, Cys, Gln, Glu, Gly, His, Ile, Leu, Lys, Met, Phe, Pro, Ser, Trp, Tyr und Val.

6. Nitrilase nach einem der vorstehenden Ansprüche, welche nach einer Temperaturbehandlung, bei der die Aktivität der Nitrilase gemäß Seq ID No: 2 mindestens um 40% reduziert wird, im Vergleich zur Nitrilase gemäß Seq ID No: 2 eine um mindestens 10% erhöhte Restaktivität besitzt im Hinblick auf die Umsetzung eines Nitrils zur entsprechenden Carbonsäure, wobei das Nitril ausgewählt ist aus der Gruppe bestehend aus 2-Methylglutaronitril, 1-(Cyanomethyl)cyclohexan-1-carbonitril und Benzonitril.

7. Nitrilase umfassend eine Aminosäuresequenz, welche eine Identität von mehr als 95% zur Aminosäuresequenz gemäß Seq ID No: 8 aufweist.

8. Verfahren zur Herstellung einer Carbonsäure aus Nitrilen durch in Kontaktbringen eines Nitrils mit einer Nitrilase gemäß einem der vorstehenden Ansprüche.

9. Verfahren nach Anspruch 8 zur Herstellung einer Carbonsäure ausgewählt aus der Gruppe bestehend aus 4-Cyano-Pentansäure, 3-Hydroxyvalerinsäure und Glykolsäure.

## Claims

1. A nitrilase comprising an amino acid sequence having at least 60% identity with the amino acid sequence according to Seq ID No: 2, which, in comparison with the nitrilase according to Seq ID No: 2,
(i) has an activity increased by at least 15% in the reaction of a nitrile to the corresponding carboxylic acid, wherein the nitrile is selected from the group consisting of 2-methylglutaronitrile, 1-(cyanomethyl)cyclohexane-1-carbonitrile and benzonitrile;
and
(ii) has at least one amino acid substitution selected from the group consisting of:
a) substitution of Leu in the position which corresponds to position 9 of Seq ID No: 2 by an amino acid selected from the group consisting of Ala, Arg, Asn, Asp, Cys, Gln, Glu, Gly, His, Ile, Lys, Met, Phe, Pro, Ser, Thr, Trp, Tyr and Val;
b) substitution of Val in the position which corresponds to position 63 of Seq ID No: 2 by an amino acid selected from the group consisting of Ala, Arg, Asn, Asp, Cys, Gln, Glu, Gly, His, Ile, Leu, Lys, Met, Phe, Pro, Ser, Thr, Trp and Tyr;
c) substitution of Thr in the position which corresponds to position 70 of Seq ID No: 2 by an amino acid selected from the group consisting of Ala, Arg, Asn, Asp, Cys, Gln, Glu, Gly, His, Ile, Leu, Lys, Met, Phe, Pro, Ser, Trp, Tyr and Val;
d) substitution of Arg in the position which corresponds to position 94 of Seq ID No: 2 by an amino acid selected from the group consisting of Ala, Asn, Asp, Cys, Gln, Glu, Gly, His, Ile, Leu, Lys, Met, Phe, Pro, Ser, Thr, Trp, Tyr and Val;
e) substitution of Leu in the position which corresponds to position 194 of Seq ID No: 2 by an amino acid selected from the group consisting of Ala, Arg, Asn, Asp, Cys, Gln, Glu, Gly, His, Ile, Lys, Met, Phe, Pro, Ser, Thr, Trp, Tyr and Val;
f) substitution of Thr in the position which corresponds to position 208 of Seq ID No: 2 by an amino acid selected from the group consisting of Ala, Arg, Asn, Asp, Cys, Gln, Glu, Gly, His, Ile, Leu, Lys, Met, Phe, Pro, Ser, Trp, Tyr and Val;
g) substitution of Cys in the position which corresponds to position 250 of Seq ID No: 2 by an amino acid selected from the group consisting of Ala, Arg, Asn, Asp, Gln, Glu, Gly, His, Ile, Leu, Lys, Met, Phe, Pro, Ser, Thr, Trp, Tyr and Val;
h) substitution of Val in the position which corresponds to position 305 of Seq ID No: 2 by an amino acid selected from the group consisting of Ala, Arg, Asn, Asp, Cys, Gln, Glu, Gly, His, Ile, Leu, Lys, Met, Phe, Pro, Ser, Thr, Trp and Tyr; and
i) substitution of Asp in the position which corresponds to position 308 of Seq ID No: 2 by an amino acid selected from the group consisting of Ala, Arg, Asn, Cys, Gln, Glu, Gly, His, Ile, Leu, Lys, Met, Phe, Pro, Ser, Thr, Trp, Tyr and Val.

2. The nitrilase as claimed in claim 1, which has at least one amino acid substitution selected from the group consisting of:
a) substitution of Leu in the position which corresponds to position 9 of Seq ID No: 2 by Lys, Glu, Ile or Arg;
b) substitution of Val in the position which corresponds to position 63 of Seq ID No: 2 by Pro, Ile, Met or Thr;
c) substitution of Thr in the position which corresponds to position 70 of Seq ID No: 2 by Ile, Ala, Cys, Asp, Glu, Phe, Gly, Leu, Met, Asn, Pro, Gln, Ser, Tyr; Arg or Val;
d) substitution of Arg in the position which corresponds to position 94 of Seq ID No: 2 by Gln, Glu, Phe, Gly, Lys, Met, Asn, Pro, Ala, Gly, His, Ile, Leu, Ser, Thr, Val or Tyr;
e) substitution of Leu in the position which corresponds to position 194 of Seq ID No: 2 by Tyr, Cys, Lys or Met;
f) substitution of Thr in the position which corresponds to position 208 of Seq ID No: 2 by Ala, Gly or Leu;
g) substitution of Cys in the position which corresponds to position 250 of Seq ID No: 2 by Gly, Ala, Phe, Lys, Met, Arg, Ser, Thr, Tyr or His;
h) substitution of Val in the position which corresponds to position 305 of Seq ID No: 2 by Leu or Met; and
i) substitution of Asp in the position which corresponds to position 308 of Seq ID No: 2 by Asn or Trp.

3. The nitrilase as claimed in claim 1 or 2, which has at least two amino acid substitutions selected from the group consisting of a), b), c), d), e), f), g), h) and i).

4. The nitrilase as claimed in any one of the preceding claims, which has at least three amino acid substitutions selected from the group consisting of a), b), c), d), e), f), g), h) and i).

5. The nitrilase as claimed in any one of the preceding claims, which additionally has at least one further amino acid substitution selected from the group consisting of:
j) substitution of Tyr in the position which corresponds to position 65 of Seq ID No: 2 by an amino acid selected from the group consisting of Ala, Arg, Asn, Asp, Cys, Gln, Glu, Gly, His, Ile, Leu, Lys, Met, Phe, Pro, Ser, Thr, Trp and Val;
k) substitution of Phe in the position which corresponds to position 168 of Seq ID No: 2 by an amino acid selected from the group consisting of Ala, Arg, Asn, Asp, Cys, Gln, Glu, Gly, His, Ile, Leu, Lys, Met, Pro, Ser, Thr, Trp, Tyr and Val;
I) substitution of Phe in the position which corresponds to position 174 of Seq ID No: 2 by an amino acid selected from the group consisting of Ala, Arg, Asn, Asp, Cys, Gln, Glu, Gly, His, Ile, Leu, Lys, Met, Pro, Ser, Thr, Trp, Tyr and Val;
m) substitution of Ile in the position which corresponds to position 201 of Seq ID No: 2 by an amino acid selected from the group consisting of Ala, Arg, Asn, Asp, Cys, Gln, Glu, Gly, His, Leu, Lys, Met, Phe, Pro, Ser, Thr, Trp, Tyr and Val; and
n) substitution of Thr in the position which corresponds to position 210 of Seq ID No: 2 by an amino acid selected from the group consisting of Ala, Arg, Asn, Asp, Cys, Gln, Glu, Gly, His, Ile, Leu, Lys, Met, Phe, Pro, Ser, Trp, Tyr and Val.

6. The nitrilase as claimed in any one of the preceding claims, which, after a temprature treatment in which the activity of the nitrilase according to Seq ID No: 2 is reduced at least by 40% in comparison with the nitrilase according to Seq ID No: 2, has a residual activity increased by at least 10% with respect to the reaction of a nitrile to the corresponding carboxylic acid, wherein the nitrile is selected from the group consisting of 2-methylglutaronitrile, 1-(cyanomethyl)cyclohexane-1-carbonitrile and benzonitrile.

7. A nitrilase comprising an amino acid sequence having an identity of greater than 95% with the amino acid sequence according to Seq ID No: 8.

8. A method for producing a carboxylic acid from nitriles by contacting a nitrile with a nitrilase as claimed in any one of the preceding claims.

9. The method as claimed in claim 8 for producing a carboxylic acid selected from the group consisting of 4-cyanopentanoic acid, 3-hydroxyvaleric acid and glycolic acid.

## Revendications

1. Nitrilase comprenant une séquence d'acides aminés ayant une identité d'au moins 60 % avec la séquence d'acides aminés selon SEQ ID NO:2, qui par comparaison avec la nitrilase selon SEQ ID NO:2
(i) présente une activité augmentée d'au moins 15 % lors de la conversion d'un nitrile en l'acide carboxylique correspondant, le nitrile étant choisi dans le groupe consistant en le 2-méthylglutaronitrile, le 1-(cyanométhyl)cyclohexane-1-carbonitrile et le benzonitrile ;
et
(ii) présente au moins une substitution d'acide aminé choisie dans le groupe consistant en :
a) la substitution de Leu sur la position qui correspond à la position 9 de SEQ ID NO:2 par un acide aminé choisi dans le groupe consistant en Ala, Arg, Asn, Asp, Cys, Gln, Glu, Gly, His, Ile, Lys, Met, Phe, Pro, Ser, Thr, Trp, Tyr et Val ;
b) la substitution de Val sur la position qui correspond à la position 63 de SEQ ID NO:2 par un acide aminé choisi dans le groupe consistant en Ala, Arg, Asn, Asp, Cys, Gln, Glu, Gly, His, Ile, Leu, Lys, Met, Phe, Pro, Ser, Thr, Trp et Tyr ;
c) la substitution de Thr sur la position qui correspond à la position 70 de SEQ ID NO:2 par un acide aminé choisi dans le groupe consistant en Ala, Arg, Asn, Asp, Cys, Gln, Glu, Gly, His, Ile, Leu, Lys, Met, Phe, Pro, Ser, Trp, Tyr et Val ;
d) la substitution de Arg sur la position qui correspond à la position 94 de SEQ ID NO:2 par un acide aminé choisi dans le groupe consistant en Ala, Asn, Asp, Cys, Gln, Glu, Gly, His, Ile, Leu, Lys, Met, Phe, Pro, Ser, Thr, Trp, Tyr et Val ;
e) la substitution de Leu sur la position qui correspond à la position 194 de SEQ ID NO:2 par un acide aminé choisi dans le groupe consistant en Ala, Arg, Asn, Asp, Cys, Gln, Glu, Gly, His, Ile, Lys, Met, Phe, Pro, Ser, Thr, Trp, Tyr et Val ;
f) la substitution de Thr sur la position qui correspond à la position 208 de SEQ ID NO:2 par un acide aminé choisi dans le groupe consistant en Ala, Arg, Asn, Asp, Cys, Gln, Glu, Gly, His, Ile, Leu, Lys, Met, Phe, Pro, Ser, Trp, Tyr et Val ;
g) la substitution de Cys sur la position qui correspond à la position 250 de SEQ ID NO:2 par un acide aminé choisi dans le groupe consistant en Ala, Arg, Asn, Asp, Gln, Glu, Gly, His, Ile, Leu, Lys, Met, Phe, Pro, Ser, Thr, Trp, Tyr et Val ;
h) la substitution de Val sur la position qui correspond à la position 305 de SEQ ID NO:2 par un acide aminé choisi dans le groupe consistant en Ala, Arg, Asn, Asp, Cys, Gln, Glu, Gly, His, Ile, Leu, Lys, Met, Phe, Pro, Ser, Thr, Trp et Tyr ; et
i) la substitution de Asp sur la position qui correspond à la position 308 de SEQ ID NO:2 par un acide aminé choisi dans le groupe consistant en Ala, Arg, Asn, Cys, Gln, Glu, Gly, His, Ile, Leu, Lys, Met, Phe, Pro, Ser, Thr, Trp, Tyr et Val.

2. Nitrilase selon la revendication 1, qui présente une substitution d'acide aminé choisie dans le groupe consistant en :
a) la substitution de Leu sur la position qui correspond à la position 9 de SEQ ID NO:2 par Lys, Glu, Ile ou Arg ;
b) la substitution de Val sur la position qui correspond à la position 63 de SEQ ID NO:2 par Pro, Ile, Met ou Thr ;
c) la substitution de Thr sur la position qui correspond à la position 70 de SEQ ID NO:2 par Ile, Ala, Cys, Asp, Glu, Phe, Gly, Leu, Met, Asn, Pro, Gln, Ser, Tyr, Arg ou Val ;
d) la substitution de Arg sur la position qui correspond à la position 94 de SEQ ID NO:2 par Gln, Glu, Phe, Gly, Lys, Met, Asn, Pro, Ala, Gly, His, Ile, Leu, Ser, Thr, Val ou Tyr ;
e) la substitution de Leu sur la position qui correspond à la position 194 de SEQ ID NO:2 par Tyr, Cys, Lys ou Met ;
f) la substitution de Thr sur la position qui correspond à la position 208 de SEQ ID NO:2 par Ala, Gly ou Leu ;
g) la substitution de Cys sur la position qui correspond à la position 250 de SEQ ID NO:2 par Gly, Ala, Phe, Lys, Met, Arg, Ser, Thr, Tyr ou His ;
h) la substitution de Val sur la position qui correspond à la position 305 de SEQ ID NO:2 par Leu ou Met ; et
i) la substitution de Asp sur la position qui correspond à la position 308 de SEQ ID NO:2 par Asn ou Trp.

3. Nitrilase selon la revendication 1 ou 2, qui comprend au moins deux substitutions d'acides aminés choisies dans le groupe consistant en a), b), c), d), e), f), g), h) et i).

4. Nitrilase selon l'une des revendications précédentes, qui comprend au moins trois substitutions d'acides aminés choisies dans le groupe consistant en a), b), c), d), e), f), g), h) et i).

5. Nitrilase selon l'une des revendications précédentes, qui comprend en outre au moins une substitution supplémentaire d'acide aminé, choisie dans le groupe consistant en :
j) la substitution de Tyr sur la position qui correspond à la position 65 de SEQ ID NO:2 par un acide aminé choisi dans le groupe consistant en Ala, Arg, Asn, Asp, Cys, Gln, Glu, Gly, His, Ile, Leu, Lys, Met, Phe, Pro, Ser, Thr, Trp et Val ;
k) la substitution de Phe sur la position qui correspond à la position 168 de SEQ ID NO:2 par un acide aminé choisi dans le groupe consistant en Ala, Arg, Asn, Asp, Cys, Gln, Glu, Gly, His, Ile, Leu, Lys, Met, Pro, Ser, Thr, Trp, Tyr et Val ;
l) la substitution de Phe sur la position qui correspond à la position 174 de SEQ ID NO:2 par un acide aminé choisi dans le groupe consistant en Ala, Arg, Asn, Asp, Cys, Gln, Glu, Gly, His, Ile, Leu, Lys, Met, Pro, Ser, Thr, Trp, Tyr et Val ;
m) la substitution de Ile sur la position qui correspond à la position 201 de SEQ ID NO:2 par un acide aminé choisi dans le groupe consistant en Ala, Arg, Asn, Asp, Cys, Gln, Glu, Gly, His, Leu, Lys, Met, Phe, Pro, Ser, Thr, Trp, Tyr et Val ; et
n) la substitution de Thr sur la position qui correspond à la position 210 de SEQ ID NO:2 par un acide aminé choisi dans le groupe consistant en Ala, Arg, Asn, Asp, Cys, Gln, Glu, Gly, His, Ile, Leu, Lys, Met, Phe, Pro, Ser, Trp, Tyr et Val.

6. Nitrilase selon l'une des revendications précédentes, qui, après un traitement thermique au cours duquel l'activité de la nitrilase selon SEQ ID NO:2 subit une réduction d'au moins 40 %, présente par comparaison avec la nitrilase selon SEQ ID NO:2 une activité résiduelle augmentée d'au moins 10 %, en liaison avec la conversion d'un nitrile en l'acide carboxylique correspondant, le nitrile étant choisi dans le groupe consistant en le 2-méthylglutaronitrile, le 1-(cyanométhyl)cyclohexane-1-carbonitrile et le benzonitrile.

7. Nitrilase comprenant une séquence d'acides aminés qui a une identité supérieure à 95 % avec la séquence d'acides aminés selon SEQ ID NO:8.

8. Procédé de préparation d'un acide carboxylique à partir de nitriles par mise en contact d'un nitrile avec une nitrilase selon l'une des revendications précédentes.

9. Procédé selon la revendication 8 pour préparer un acide carboxylique choisi dans le groupe consistant en l'acide 4-cyano-pentanoïque, l'acide 3-hydroxyvalérique et l'acide glycolique.


### SEQUENCE LISTING

<110> c-LEcta GmbH
<120> Nitrilases
<130> IV0010(EP)
<160> 8
<170> PatentIn version 3.5
<210> 1
   <211> 1110
   <212> DNA
   <213> Acidovorax facilis
<400> 1
   atggtgtctt acaactctaa atttctggcg gcgactgtgc aggcggagcc agtttggctc      60
   gacgcggatg ctacgatcga caaatctatc ggtatcatcg aagaagcggc gcagaaaggt     120
   gcgtctctga tcgctttccc ggaagtattt atccctggtt acccatactg ggcgtggctc     180
   ggcgacgtaa aatacagcct gagcttcact tctcgctacc acgaaaactc tctcgagctg     240
   ggtgatgacc gtatgcgtcg tctccaactc gctgcacgtc gcaacaaaat tgcgctggtt     300
   atgggctaca gcgagcgtga agcgggtagc cgctacctgt cccaggtttt catcgacgag     360
   cgcggtgaaa ttgtcgcgaa ccgtcgtaaa ctcaagccga cccatgttga gcgcaccatc     420
   tacggtgaag gtaacggcac ggatttcctc acccatgact tcgcgttcgg tcgtgttggt     480
   ggcctcaact gctgggagca ctttcagccg ctctccaaat tcatgatgta ctctctgggc     540
   gaacaagttc atgtcgcatc ctggcctgca atgtccccgc tgcaaccgga tgttttccag     600
   ctgtctatcg aagcgaatgc gaccgtcacc cgtagctatg ccatcgaggg tcagaccttc     660
   gtgctgtgct ctacgcaggt tatcggtccg tctgcaatcg agacgttctg cctgaatgac     720
   gaacagcgtg cgctgctgcc gcagggttgt ggttgggcgc gtatctatgg cccggatggt     780
   tccgaactgg caaaacctct cgcagaagat gcggagggta tcctgtatgc ggaaatcgac     840
   ctggaacaga ttctcctcgc aaaagcaggc gctgacccgg ttggccacta ttctcgtcca     900
   gatgttctca gcgttcagtt cgacccacgt aaccataccc cggttcatcg tatcggcatt     960
   gatggccgcc tggatgtaaa cacccgttcc cgtgtagaaa actttcgcct ccgccaggct    1020
   gctgagcagg agcgtcaggc gtccaaacgc ctgggcacca agctctttga acagtctctg    1080
   ctcgccgaag agccggtgcc ggctaaatga                                     1110
<210> 2
   <211> 369
   <212> PRT
   <213> Acidovorax facilis
<400> 2
   Met Val Ser Tyr Asn Ser Lys Phe Leu Ala Ala Thr Val Gln Ala Glu
   1 5 10 15
   Pro Val Trp Leu Asp Ala Asp Ala Thr Ile Asp Lys Ser Ile Gly Ile
   20 25 30
   Ile Glu Glu Ala Ala Gln Lys Gly Ala Ser Leu Ile Ala Phe Pro Glu
   35 40 45
   Val Phe Ile Pro Gly Tyr Pro Tyr Trp Ala Trp Leu Gly Asp Val Lys
   50 55 60
   Tyr Ser Leu Ser Phe Thr Ser Arg Tyr His Glu Asn Ser Leu Glu Leu
   65 70 75 80
   Gly Asp Asp Arg Met Arg Arg Leu Gln Leu Ala Ala Arg Arg Asn Lys
   85 90 95
   Ile Ala Leu Val Met Gly Tyr Ser Glu Arg Glu Ala Gly Ser Arg Tyr
   100 105 110
   Leu Ser Gln Val Phe Ile Asp Glu Arg Gly Glu Ile Val Ala Asn Arg
   115 120 125
   Arg Lys Leu Lys Pro Thr His Val Glu Arg Thr Ile Tyr Gly Glu Gly
   130 135 140
   Asn Gly Thr Asp Phe Leu Thr His Asp Phe Ala Phe Gly Arg Val Gly
   145 150 155 160
   Gly Leu Asn Cys Trp Glu His Phe Gln Pro Leu Ser Lys Phe Met Met
   165 170 175
   Tyr Ser Leu Gly Glu Gln Val His Val Ala Ser Trp Pro Ala Met Ser
   180 185 190
   Pro Leu Gln Pro Asp Val Phe Gln Leu Ser Ile Glu Ala Asn Ala Thr
   195 200 205
   Val Thr Arg Ser Tyr Ala Ile Glu Gly Gln Thr Phe Val Leu Cys Ser
   210 215 220
   Thr Gln Val Ile Gly Pro Ser Ala Ile Glu Thr Phe Cys Leu Asn Asp
   225 230 235 240
   Glu Gln Arg Ala Leu Leu Pro Gln Gly Cys Gly Trp Ala Arg Ile Tyr
   245 250 255
   Gly Pro Asp Gly Ser Glu Leu Ala Lys Pro Leu Ala Glu Asp Ala Glu
               260                 265                 270
   Gly Ile Leu Tyr Ala Glu Ile Asp Leu Glu Gln Ile Leu Leu Ala Lys
           275                 280                 285
   Ala Gly Ala Asp Pro Val Gly His Tyr Ser Arg Pro Asp Val Leu Ser
       290                 295                 300
   Val Gln Phe Asp Pro Arg Asn His Thr Pro Val His Arg Ile Gly Ile
   305                 310                 315                 320
   Asp Gly Arg Leu Asp Val Asn Thr Arg Ser Arg Val Glu Asn Phe Arg
                   325                 330                 335
   Leu Arg Gln Ala Ala Glu Gln Glu Arg Gln Ala Ser Lys Arg Leu Gly
               340                 345                 350
   Thr Lys Leu Phe Glu Gln Ser Leu Leu Ala Glu Glu Pro Val Pro Ala
           355                 360                 365
   Lys
<210> 3
   <211> 369
   <212> PRT
   <213> Unknown
<220>
   <223> chemically synthesized
<400> 3
   Met Val Ser Tyr Asn Ser Lys Phe Leu Ala Ala Thr Val Gln Ala Glu
   1               5                   10                  15
   Pro Val Trp Leu Asp Ala Asp Ala Thr Ile Asp Lys Ser Ile Gly Ile
               20                  25                  30
   Ile Glu Glu Ala Ala Gln Lys Gly Ala Ser Leu Ile Ala Phe Pro Glu
           35                  40                  45
   Val Phe Ile Pro Gly Tyr Pro Tyr Trp Ala Trp Leu Gly Asp Pro Lys
       50                  55                  60
   Tyr Ser Leu Ser Phe Thr Ser Arg Tyr His Glu Asn Ser Leu Glu Leu
   65                  70                  75                  80
   Gly Asp Asp Arg Met Arg Arg Leu Gln Leu Ala Ala Arg Arg Asn Lys
                   85                  90                  95
   Ile Ala Leu Val Met Gly Tyr Ser Glu Arg Glu Ala Gly Ser Arg Tyr
               100                 105                 110
   Leu Ser Gln Val Phe Ile Asp Glu Arg Gly Glu Ile Val Ala Asn Arg
           115                 120                 125
   Arg Lys Leu Lys Pro Thr His Val Glu Arg Thr Ile Tyr Gly Glu Gly
       130                 135                 140
   Asn Gly Thr Asp Phe Leu Thr His Asp Phe Ala Phe Gly Arg Val Gly
   145                 150                 155                 160
   Gly Leu Asn Cys Trp Glu His Phe Gln Pro Leu Ser Lys Phe Met Met
                   165                 170                 175
   Tyr Ser Leu Gly Glu Gln Val His Val Ala Ser Trp Pro Ala Met Ser
               180                 185                 190
   Pro Leu Gln Pro Asp Val Phe Gln Leu Ser Ile Glu Ala Asn Ala Thr
           195                 200                 205
   Val Thr Arg Ser Tyr Ala Ile Glu Gly Gln Thr Phe Val Leu Cys Ser
       210                 215                 220
   Thr Gln Val Ile Gly Pro Ser Ala Ile Glu Thr Phe Cys Leu Asn Asp
   225                 230                 235                 240
   Glu Gln Arg Ala Leu Leu Pro Gln Gly Gly Gly Trp Ala Arg Ile Tyr
                   245                 250                 255
   Gly Pro Asp Gly Ser Glu Leu Ala Lys Pro Leu Ala Glu Asp Ala Glu
               260                 265                 270
   Gly Ile Leu Tyr Ala Glu Ile Asp Leu Glu Gln Ile Leu Leu Ala Lys
           275                 280                 285
   Ala Gly Ala Asp Pro Val Gly His Tyr Ser Arg Pro Asp Val Leu Ser
       290                 295                 300
   Val Gln Phe Asp Pro Arg Asn His Thr Pro Val His Arg Ile Gly Ile
   305                 310                 315                 320
   Asp Gly Arg Leu Asp Val Asn Thr Arg Ser Arg Val Glu Asn Phe Arg
                   325                 330                 335
   Leu Arg Gln Ala Ala Glu Gln Glu Arg Gln Ala Ser Lys Arg Leu Gly
   340 345 350
   Thr Lys Leu Phe Glu Gln Ser Leu Leu Ala Glu Glu Pro Val Pro Ala
   355 360 365
   Lys
<210> 4
   <211> 369
   <212> PRT
   <213> Unknown
<220>
   <223> chemically synthesized
<400> 4
   Met Val Ser Tyr Asn Ser Lys Phe Leu Ala Ala Thr Val Gln Ala Glu
   1 5 10 15
   Pro Val Trp Leu Asp Ala Asp Ala Thr Ile Asp Lys Ser Ile Gly Ile
   20 25 30
   Ile Glu Glu Ala Ala Gln Lys Gly Ala Ser Leu Ile Ala Phe Pro Glu
   35 40 45
   Val Phe Ile Pro Gly Tyr Pro Tyr Trp Ala Trp Leu Gly Asp Val Lys
   50 55 60
   Tyr Ser Leu Ser Phe Thr Ser Arg Tyr His Glu Asn Ser Leu Glu Leu
   65 70 75 80
   Gly Asp Asp Arg Met Arg Arg Leu Gln Leu Ala Ala Arg Arg Asn Lys
   85 90 95
   Ile Ala Leu Val Met Gly Tyr Ser Glu Arg Glu Ala Gly Ser Arg Tyr
   100 105 110
   Leu Ser Gln Val Phe Ile Asp Glu Arg Gly Glu Ile Val Ala Asn Arg
   115 120 125
   Arg Lys Leu Lys Pro Thr His Val Glu Arg Thr Ile Tyr Gly Glu Gly
   130 135 140
   Asn Gly Thr Asp Phe Leu Thr His Asp Phe Ala Phe Gly Arg Val Gly
   145 150 155 160
   Gly Leu Asn Cys Trp Glu His Val Gln Pro Leu Ser Lys Phe Met Met
                   165                 170                 175
   Tyr Ser Leu Gly Glu Gln Val His Val Ala Ser Trp Pro Ala Met Ser
               180                 185                 190
   Pro Leu Gln Pro Asp Val Phe Gln Leu Ser Ile Glu Ala Asn Ala Thr
           195                 200                 205
   Val Thr Arg Ser Tyr Ala Ile Glu Gly Gln Thr Phe Val Leu Cys Ser
       210                 215                 220
   Thr Gln Val Ile Gly Pro Ser Ala Ile Glu Thr Phe Cys Leu Asn Asp
   225                 230                 235                 240
   Glu Gln Arg Ala Leu Leu Pro Gln Gly Gly Gly Trp Ala Arg Ile Tyr
                   245                 250                 255
   Gly Pro Asp Gly Ser Glu Leu Ala Lys Pro Leu Ala Glu Asp Ala Glu
               260                 265                 270
   Gly Ile Leu Tyr Ala Glu Ile Asp Leu Glu Gln Ile Leu Leu Ala Lys
           275                 280                 285
   Ala Gly Ala Asp Pro Val Gly His Tyr Ser Arg Pro Asp Val Leu Ser
       290                 295                 300
   Val Gln Phe Asp Pro Arg Asn His Thr Pro Val His Arg Ile Gly Ile
   305                 310                 315                 320
   Asp Gly Arg Leu Asp Val Asn Thr Arg Ser Arg Val Glu Asn Phe Arg
                   325                 330                 335
   Leu Arg Gln Ala Ala Glu Gln Glu Arg Gln Ala Ser Lys Arg Leu Gly
               340                 345                 350
   Thr Lys Leu Phe Glu Gln Ser Leu Leu Ala Glu Glu Pro Val Pro Ala
           355                 360                 365
   Lys
<210> 5
   <211> 369
   <212> PRT
   <213> Unknown
<220>
   <223> chemically synthesized
<400> 5
   Met Val Ser Tyr Asn Ser Lys Phe Leu Ala Ala Thr Val Gln Ala Glu
   1 5 10 15
   Pro Val Trp Leu Asp Ala Asp Ala Thr Ile Asp Lys Ser Ile Gly Ile
   20 25 30
   Ile Glu Glu Ala Ala Gln Lys Gly Ala Ser Leu Ile Ala Phe Pro Glu
   35 40 45
   Val Phe Ile Pro Gly Tyr Pro Tyr Trp Ala Trp Leu Gly Asp Pro Lys
   50 55 60
   Tyr Ser Leu Ser Phe Thr Ser Arg Tyr His Glu Asn Ser Leu Glu Leu
   65 70 75 80
   Gly Asp Asp Arg Met Arg Arg Leu Gln Leu Ala Ala Arg Arg Asn Lys
   85 90 95
   Ile Ala Leu Val Met Gly Tyr Ser Glu Arg Glu Ala Gly Ser Arg Tyr
   100 105 110
   Leu Ser Gln Val Phe Ile Asp Glu Arg Gly Glu Ile Val Ala Asn Arg
   115 120 125
   Arg Lys Leu Lys Pro Thr His Val Glu Arg Thr Ile Tyr Gly Glu Gly
   130 135 140
   Asn Gly Thr Asp Phe Leu Thr His Asp Phe Ala Phe Gly Arg Val Gly
   145 150 155 160
   Gly Leu Asn Cys Trp Glu His Val Gln Pro Leu Ser Lys Phe Met Met
   165 170 175
   Tyr Ser Leu Gly Glu Gln Val His Val Ala Ser Trp Pro Ala Met Ser
   180 185 190
   Pro Leu Gln Pro Asp Val Phe Gln Leu Ser Ile Glu Ala Asn Ala Thr
   195 200 205
   Val Thr Arg Ser Tyr Ala Ile Glu Gly Gln Thr Phe Val Leu Cys Ser
   210 215 220
   Thr Gln Val Ile Gly Pro Ser Ala Ile Glu Thr Phe Cys Leu Asn Asp
   225 230 235 240
   Glu Gln Arg Ala Leu Leu Pro Gln Gly Cys Gly Trp Ala Arg Ile Tyr
                   245                 250                 255
   Gly Pro Asp Gly Ser Glu Leu Ala Lys Pro Leu Ala Glu Asp Ala Glu
               260                 265                 270
   Gly Ile Leu Tyr Ala Glu Ile Asp Leu Glu Gln Ile Leu Leu Ala Lys
           275                 280                 285
   Ala Gly Ala Asp Pro Val Gly His Tyr Ser Arg Pro Asp Val Leu Ser
       290                 295                 300
   Val Gln Phe Asp Pro Arg Asn His Thr Pro Val His Arg Ile Gly Ile
   305                 310                 315                 320
   Asp Gly Arg Leu Asp Val Asn Thr Arg Ser Arg Val Glu Asn Phe Arg
                   325                 330                 335
   Leu Arg Gln Ala Ala Glu Gln Glu Arg Gln Ala Ser Lys Arg Leu Gly
               340                 345                 350
   Thr Lys Leu Phe Glu Gln Ser Leu Leu Ala Glu Glu Pro Val Pro Ala
           355                 360                 365
   Lys
<210> 6
   <211> 369
   <212> PRT
   <213> Unknown
<220>
   <223> chemically synthesized
<400> 6
   Met Val Ser Tyr Asn Ser Lys Phe Leu Ala Ala Thr Val Gln Ala Glu
   1               5                   10                  15
   Pro Val Trp Leu Asp Ala Asp Ala Thr Ile Asp Lys Ser Ile Gly Ile
               20                  25                  30
   Ile Glu Glu Ala Ala Gln Lys Gly Ala Ser Leu Ile Ala Phe Pro Glu
           35                  40                  45
   Val Phe Ile Pro Gly Tyr Pro Tyr Trp Ala Trp Leu Gly Asp Pro Lys
       50                  55                  60
   Tyr Ser Leu Ser Phe Thr Ser Arg Tyr His Glu Asn Ser Leu Glu Leu
   65                  70                  75                  80
   Gly Asp Asp Arg Met Arg Arg Leu Gln Leu Ala Ala Arg Arg Asn Lys
                   85                  90                  95
   Ile Ala Leu Val Met Gly Tyr Ser Glu Arg Glu Ala Gly Ser Arg Tyr
               100                 105                 110
   Leu Ser Gln Val Phe Ile Asp Glu Arg Gly Glu Ile Val Ala Asn Arg
           115                 120                 125
   Arg Lys Leu Lys Pro Thr His Val Glu Arg Thr Ile Tyr Gly Glu Gly
       130                 135                 140
   Asn Gly Thr Asp Phe Leu Thr His Asp Phe Ala Phe Gly Arg Val Gly
   145                 150                 155                 160
   Gly Leu Asn Cys Trp Glu His Val Gln Pro Leu Ser Lys Phe Met Met
                   165                 170                 175
   Tyr Ser Leu Gly Glu Gln Val His Val Ala Ser Trp Pro Ala Met Ser
               180                 185                 190
   Pro Leu Gln Pro Asp Val Phe Gln Leu Ser Ile Glu Ala Asn Ala Thr
           195                 200                 205
   Val Thr Arg Ser Tyr Ala Ile Glu Gly Gln Thr Phe Val Leu Cys Ser
       210                 215                 220
   Thr Gln Val Ile Gly Pro Ser Ala Ile Glu Thr Phe Cys Leu Asn Asp
   225                 230                 235                 240
   Glu Gln Arg Ala Leu Leu Pro Gln Gly Gly Gly Trp Ala Arg Ile Tyr
                   245                 250                 255
   Gly Pro Asp Gly Ser Glu Leu Ala Lys Pro Leu Ala Glu Asp Ala Glu
               260                 265                 270
   Gly Ile Leu Tyr Ala Glu Ile Asp Leu Glu Gln Ile Leu Leu Ala Lys
           275                 280                 285
   Ala Gly Ala Asp Pro Val Gly His Tyr Ser Arg Pro Asp Val Leu Ser
       290                 295                 300
   Val Gln Phe Asp Pro Arg Asn His Thr Pro Val His Arg Ile Gly Ile
   305                 310                 315                 320
   Asp Gly Arg Leu Asp Val Asn Thr Arg Ser Arg Val Glu Asn Phe Arg
   325 330 335
   Leu Arg Gln Ala Ala Glu Gln Glu Arg Gln Ala Ser Lys Arg Leu Gly
   340 345 350
   Thr Lys Leu Phe Glu Gln Ser Leu Leu Ala Glu Glu Pro Val Pro Ala
   355 360 365
   Lys
<210> 7
   <211> 381
   <212> PRT
   <213> Unknown
<220>
   <223> chemically synthesized
<400> 7
   Met Leu Thr Tyr Lys Gly Val Phe Lys Ala Ala Thr Val Gln Ala Glu
   1 5 10 15
   Pro Val Trp Met Asp Ala Asp Ala Thr Ile Thr Lys Ala Ile Arg Ile
   20 25 30
   Ile Glu Glu Ala Ala Asp Asn Gly Ala Lys Phe Val Ala Phe Pro Glu
   35 40 45
   Val Phe Ile Pro Gly Tyr Pro Trp Trp Ile Trp Leu Gly Thr Ala Met
   50 55 60
   Trp Gly Ala Lys Phe Val Val Pro Phe His Glu Asn Cys Leu Glu Leu
   65 70 75 80
   Gly Asp Lys Arg Met Gln Arg Ile Gln Ala Ala Ala Lys Gln Asn Gly
   85 90 95
   Ile Ala Leu Val Met Gly Tyr Gly Glu Arg Asp Gly Gly Ser Arg Tyr
   100 105 110
   Met Ser Gln Val Phe Ile Asp Asp Ser Gly Lys Ile Val Ala Asn Arg
   115 120 125
   Arg Lys Leu Lys Pro Thr His Glu Glu Arg Thr Ile Phe Gly Glu Gly
   130 135 140
   Asn Gly Ser Asp Phe Ile Thr His Asp Phe Pro Phe Ala Arg Val Gly
   145                 150                 155                 160
   Gly Phe Asn Cys Trp Glu His Leu Gln Pro Leu Ser Lys Tyr Met Met
                   165                 170                 175
   Tyr Ser Leu Gln Glu Gln Val His Val Ala Ser Trp Pro Ala Met Cys
               180                 185                 190
   Thr Tyr Gln Pro Asp Val Pro Gln Leu Gly Ala Gly Ala Asn Glu Ala
           195                 200                 205
   Val Thr Arg Ser Tyr Ala Ile Glu Gly Ala Cys Tyr Val Leu Gly Ala
       210                 215                 220
   Thr Leu Val Ile Gly Lys Ala Ala His Asp Ala Phe Cys Asp Thr Glu
   225                 230                 235                 240
   Glu His His Lys Leu Leu Gly Met Gly Gly Gly Trp Ala Arg Ile Phe
                   245                 250                 255
   Gly Pro Asp Gly Glu Tyr Leu Ala Glu Ser Leu Ala His Asp Ala Glu
               260                 265                 270
   Gly Ile Leu Tyr Ala Asp Ile Asp Leu Ser Lys Ile Leu Leu Ala Lys
           275                 280                 285
   Ala Asn Thr Asp Thr Val Gly His Tyr Ala Arg Pro Asp Val Leu Ser
       290                 295                 300
   Leu Leu Val Asn Thr His Asn Pro Gly Pro Val Arg Tyr Leu Asp Glu
   305                 310                 315                 320
   Glu Gly Arg Gln Val Ser Thr Ser Ile Arg Arg His Glu Lys Leu Glu
                   325                 330                 335
   Gly Gln Ser Leu Asp Leu Glu Val Thr Pro Ala Thr Pro Ala Thr Leu
               340                 345                 350
   Asp Ile Ala Ser Leu Val Gln Gln Ala Lys Pro Ser Thr Val Lys Ser
           355                 360                 365
   Glu Ser Asn Ala Ser Thr Lys Gln Pro Asp Leu Ala Val
       370                 375                 380
<210> 8
   <211> 369
   <212> PRT
   <213> Unknown
<220>
   <223> chemically synthesized
<400> 8
   Met Leu Thr Tyr Lys Gly Val Phe Lys Ala Ala Thr Val Gln Ala Glu
   1               5                   10                  15
   Pro Val Trp Met Asp Ala Asp Ala Thr Ile Thr Lys Ala Ile Arg Ile
               20                  25                  30
   Ile Glu Glu Ala Ala Asp Asn Gly Ala Lys Phe Val Ala Phe Pro Glu
           35                  40                  45
   Val Phe Ile Pro Gly Tyr Pro Tyr Trp Ala Trp Leu Gly Asp Pro Lys
       50                  55                  60
   Tyr Ser Leu Ser Phe Thr Ser Arg Tyr His Glu Asn Ser Leu Glu Leu
   65                  70                  75                  80
   Gly Asp Asp Arg Met Arg Arg Leu Gln Leu Ala Ala Arg Gln Asn Lys
                   85                  90                  95
   Ile Ala Leu Val Met Gly Tyr Ser Glu Arg Glu Ala Gly Ser Arg Tyr
               100                 105                 110
   Leu Ser Gln Val Phe Ile Asp Glu Arg Gly Glu Ile Val Ala Asn Arg
           115                 120                 125
   Arg Lys Leu Lys Pro Thr His Val Glu Arg Thr Ile Tyr Gly Glu Gly
       130                 135                 140
   Asn Gly Thr Asp Phe Leu Thr His Asp Phe Ala Phe Gly Arg Val Gly
   145                 150                 155                 160
   Gly Leu Asn Cys Trp Glu His Val Gln Pro Leu Ser Lys Phe Met Met
                   165                 170                 175
   Tyr Ser Leu Gly Glu Gln Val His Val Ala Ser Trp Pro Ala Met Ser
               180                 185                 190
   Pro Tyr Gln Pro Asp Val Phe Gln Leu Ser Ile Glu Ala Asn Ala Ala
           195                 200                 205
   Val Thr Arg Ser Tyr Ala Ile Glu Gly Gln Thr Phe Val Leu Cys Ser
       210                 215                 220
   Thr Gln Val Ile Gly Pro Ser Ala Ile Glu Thr Phe Cys Leu Asn Asp
   225                 230                 235                 240
   Glu Gln Arg Ala Leu Leu Pro Gln Gly Gly Gly Trp Ala Arg Ile Tyr
                   245                 250                 255
   Gly Pro Asp Gly Ser Glu Leu Ala Lys Pro Leu Ala Glu Asp Ala Glu
               260                 265                 270
   Gly Ile Leu Tyr Ala Glu Ile Asp Leu Glu Gln Ile Leu Leu Ala Lys
           275                 280                 285
   Ala Gly Ala Asp Pro Val Gly His Tyr Ser Arg Pro Asp Val Leu Ser
       290                 295                 300
   Leu Gln Phe Asp Pro Arg Asn His Thr Pro Val His Arg Ile Gly Ile
   305                 310                 315                 320
   Asp Gly Arg Leu Asp Val Asn Thr Arg Ser Arg Val Glu Asn Phe Arg
                   325                 330                 335
   Leu Arg Gln Ala Ala Glu Gln Glu Arg Gln Ala Ser Lys Arg Leu Gly
               340                 345                 350
   Thr Lys Leu Phe Glu Gln Ser Leu Leu Ala Glu Glu Pro Val Pro Ala
           355                 360                 365
   Lys